(19) Europäisches Patentamt

European Patent Office

Office européen des brevets

(11) Veröffentlichungsnummer: **0 089 529**
**B1**

(12) **EUROPÄISCHE PATENTSCHRIFT**

(45) Veröffentlichungstag der Patentschrift:
22.06.88

(21) Anmeldenummer: 83102130.8

(22) Anmeldetag: 04.03.83

(51) Int. Cl.⁴: **C 08 B  37/00,** A 61 K  31/715,
**A 61 K  7/00**

(54) **Polysaccharidkonzentrate, Verfahren zu ihrer Herstellung und diese enthaltende Arzneimittel und kosmetische Präparate.**

(30) Priorität: 04.03.82  HU 66082
09.02.83  HU 66082

(43) Veröffentlichungstag der Anmeldung:
28.09.83 Patentblatt 83/39

(45) Bekanntmachung des Hinweises auf die Patenterteilung:
22.06.88 Patentblatt 88/25

(84) Benannte Vertragsstaaten:
AT BE CH DE FR GB IT LI LU NL SE

(56) Entgegenhaltungen:
DE - C - 876 305
FR - M - 2 426
GB - A - 878 430
GB - A - 2 008 408

CHEMICAL & PHARMACEUTICAL BULLETIN, Band 25,
Nr. 6, Juni 1977, Seiten 1357-1362; M. TOMODA et al:
"Plant mucilages. XVI. Isolation and characterization of
a mucous polysaccharide, "Aithaea-muciiage O", from
the roots of Althaea officinalis"
CHEMICAL ABSTRACTS, Band 91, Nr. 5, 30. Juli 1979,
Seite 315, Nr. 35698q, Columbus, Ohio, USA; S.
KOCURIK et al.: "Saccharides of camomile (Matricaria
chamomilla) flowers. II. Water-soluble polysaccharide"
& FARM. OBZ. 1979, 48(3), 111-118

(56) Entgegenhaltungen: (Fortsetzung)
**Die Akte enthält technische Angaben, die nach dem
Eingang der Anmeldung eingereicht wurden und die
nicht in dieser Patentschrift enthalten sind.**

(73) Patentinhaber: **KÖZPONTI VALTO-ES HITELBANK RT
INNOVACIOS ALAP, Szabadság tér 5-6,
H-1054 Budapest (HU)**

(72) Erfinder: **Szendrei, Káimán, Prof. Dr., Hunyadi sugár
ut 56, H-6725 Szeged (HU)**
Erfinder: **Minker, Emil, Prof. Dr., Somogyi Béla ut 6,
H-6720 Szeged (HU)**
Erfinder: **Rózsa, Zsuzsanna, Dr., Tarján széle 622. épület
III. 7, H-6723 Szeged (HU)**
Erfinder: **Koch, Lehel, Dipl. Chem., Ora u. 18/c,
H-1125 Budapest (HU)**
Erfinder: **Woif, Lajos, Dr., Németvölgyi ut 28/a,
H-1126 Budapest (HU)**

(74) Vertreter: **Beszédes, Stephan G. Dr., Münchener
Strasse 80a Postfach 1168, D-8060 Dachau (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft neue Polysaccharidkonzentrate pflanzlichen Ursprungs sowie diese enthaltende Arzneimittel und kosmetische Präparate, insbesondere solche mit entzündungshemmender Wirkung.

Unter Polysacchariden sind optisch aktive Heteropolysaccharide, die aus Monosacchariden von Furanose- oder Pyranosestruktur mit Äther- oder Glykosidbindung aufgebaut sind und deren saure oder basische Funktionen gegebenenfalls mit einem Eiweiss und/oder Peptid gekoppelt sein können (Peptidoglykane oder Glykoproteine), zu verstehen. Unter entzündungshemmender Wirkung sind alle Wirkungen, die mit dem Carraghenin-Ödem-Versuch an Rattenpfoten erfasst beziehungsweise nicht ausgeschlossen werden können, zu verstehen. Die im Carraghenin-Ödem-Versuch an Rattenpfoten aktiven entzündungshemmenden Stoffe können Antirheumatica, Mittel gegen Geschwüre beziehungsweise Ulken und äusserlich anwendbare Salben, Gele oder Lösungen, gegebenenfalls auch Heilkosmetika, sein.

Die in der Heilkunde gegenwärtig angewandten entzündungshemmenden Wirkstoffe nicht natürlichen Ursprunges enthalten als eine Möglichkeit Steroide, was zahlreiche Toleranzprobleme aufwirft. Beispielsweise haben sie häufig eine Hormonwirkung, vergrössern die Natriumretention des Organismus oder verursachen Atrophie. Die nicht-steroiden entzündungshemmenden Substanzen haben ebenfalls Nebenwirkungen, die ihre Anwendung beschränken.
1-[4'-(Chlor)-benzoyl]-5-[methoxy]-2-[3''-
(methyl)-indolyl]-essigsäure {Indometacin}
und Salicylate haben zum Beispiel die Nebenwirkung, die Widerstandskraft des Organismus zu schwächen, den Magen-Darm-Trakt anzugreifen und innere Blutungen und Magengeschwüre zu verursachen. Wegen der toxischen Nebenwirkungen richten sich die Bestrebungen immer mehr auf die Auffindung und Bereitstellung von natürlichen entzündungshemmenden Stoffen. Derartige Forschungen werden um so intensiver vorangetrieben, als die entzündungshemmenden Mittel in letzter Zeit ein grösseres Anwendungsgebiet gefunden haben, da sie auch zur Behandlung von Störungen der Sauerstoffversorgung von Geweben, zum Beispiel Herzinfarkt, sonstigen Gewebeschäden durch Kreislaufstörungen und zur Behandlung von Schockzuständen angewandt werden.

Auf den Gebieten, auf denen entzündungshemmende Stoffe erforderlich sind, werden in der Heilkunde und Kosmetik gleichermassen zahlreiche Heilpflanzen und aus diesen hergestellte Präparate verwendet. Diejenigen Bestandteile der Drogenpflanzen und der aus ihnen gewonnenen Präparate, welche für die Wirkung verantwortlich sind, sind zum grossen Teil noch unbekannt. Die Kamillenblüte (Chamomillae flos) wird in Form von Tees, Teemischungen, wässrigen oder lösungsmittelhaltigen Extrakten und Destillaten (ätherisches Öl) verbreitet zur Heilung der unterschiedlichsten Krankheiten verwendet. Zu den vermuteten und zum Teil nachgewiesenen pharmakologischen Wirkungen der Droge gehören die entzündungshemmende und die auf den Herzmuskel ausgeübte Wirkung (F. Auster, J. Schäfer: Arzneipflanzen 15. 19. 21. Lieferungen; W. Spaich: Moderne Phytotherapie, Haug Verlag 1978). Die Kamillenblüte wird in erster Linie in der Heilkunde sowie auch in der Kosmetik zur Behandlung von entzündlichen Vorgängen an der Haut und an den Schleimhäuten angewandt, und zwar in Form von Salben, Bädern, Inhalationsflüssigkeiten, Pinseln oder Tees, gegebenenfalls in Form von wässrigen Auszügen oder des in Kosmetika eingearbeiteten Wirkstoffes. Innerlich angewandt wird Kamille bei Katarrhen und Krämpfen des Verdauungssystemes, bei geschwürartigen Erkrankungen der Schleimhäute oder deren Verletzung meistens in Form von Tees. Nach neueren Forschungen ist die entzündungshemmende Wirkung der Kamille auf Terpenoide von Lipoidcharakter (Kamazulen, α-Bisabolol und Bisabololoxyde) zurückzuführen. (O. Isaak: Planta Med. 35 [1979], 118). Durch Messen der entzündungshemmenden Wirkung der Wirkungsträger konnte festgestellt werden, dass deren $ED_{50}$-Werte bei 1 465 bis 3 164 mg/kg liegen. Zwischen der tatsächlichen Wirkstoffkonzentration der Droge und der Präparate einerseits und der den Wirkstoffen zugeschriebenen Heilwirkung besteht ein augenfälliger Unterschied (Verzárné Petri G., Marczal J.: Herba Hung. 15 (2) [1976], 69). Wenn sich die als Wirkungsträger nachgewiesenen lipoidartigen Verbindungen in Wasser schlecht lösen, erhebt sich die Frage: Wieso können sie die wichtigsten Bestandteile der mit Wasser bereiteten Tees und Präparate sein? Es gibt zahlreiche als Heilpflanzen verwendete oder nicht als Heilpflanzen angesehene Pflanzenarten, aus denen die entzündungshemmenden Bestandteile nicht gewonnen werden konnten. Zu diesen gehören zum Beispiel die Arten Tilia, Malva, Plantago, Linum usitatissimum, Trigonella foenum-graecum und Cydonia oblongara.

Ferner ist aus der britischen Patentanmeldung 2 008 408 ein Mittel zum Unterdrücken von Sodbrennen durch Rücklauf des Magensaftes aus einer wässrigen Lösung eines Pflanzenpolysaccharidschleims, beispielsweise Quittensamen-Pflanzenschleim, bei welchem es sich um eine trinkfähige Flüssigkeit bei einem pH-Wert oberhalb des pH des Mageninhaltes, das jedoch ein Gel bei einem pH-Wert des menschlichen Mageninhaltes bildet, handelt, und einer physiologisch verträglichen Flüssigkeit mit geringer Dichte, wie einem Pflanzenöl, welches das spezifische Gewicht des Mittels auf einen Wert unterhalb des Wertes des menschlichen Mageninhalts herabsetzt, so dass das im Magen gebildete Gel auf dem Mageninhalt schwimmt, bekannt. Die Herstellung dieses Mittels erfolgt lediglich durch Extrahieren des Schleimes aus den Samen mit heissem Wasser und Abtrennen des wässrigen Schleimes von den Samenrückständen, während von einer Fällung einer bestimmten Fraktion keine Rede ist. Damit gelangen alle Polysaccharide in den Pflanzenschleim, so dass ein grosser Teil der in ihm enthaltenen Polysaccharide nur geringe Molekulargewichte unter 75 000 hat, denn je kleiner das Molekulargewicht ist, um so besser ist die Löslichkeit in Wasser.

Weiterhin ist in der deutschen Patentschrift 876 305 ein Verfahren zur Gewinnung therapeutisch wirksamer Pflanzenpräparate, die neben den wasserlöslichen und wasserunlöslichen Wirkstoffen auch die ätherischen Öle und Aromastoffe enthalten, bei welchem die pflanzlichen Rohstoffe nach vorheriger mechanischer Aufbereitung unter zusätzlicher Verwendung von Saponinen, Netz- und Emulgiermitteln oder ähnlich wirkenden Stoffen in eine homogene, kolloidale wässrige Lösung beziehungsweise Emulsion gebracht und beispielsweise durch Filterpassage die unlöslichen Festsubstanzen der Pflanzen vollständig oder zum grössten Teil entfernt werden, sowie die entzündungshemmende Wirkung der so erhaltenen Produkte beschrieben. Durch die Verwendung der Saponine, Netz- und Emulgiermittel oder ähnlich wirkenden Stoffe werden aber auch die wasserunlöslichen Stoffe und niedermolekularen Polysaccharide mit Molekulargewichten unter 75 000 sowie die ätherischen Öle und Aromastoffe mit niederen Molekulargewichten von etwa 700 bis 800 in den Auszug hineingebracht und, da keine Fällung einer Fraktion vorgesehen ist, bleiben sie auch in diesen. Dies hat zur Folge, dass die Produkte dieser Druckschrift nur eine geringe entzündungshemmende Wirkung mit $ED_{50}$-Werten von etwa 1 500 bis 3 000 mg/kg haben.

Ferner sind aus der französischen Patentschrift 2 426 M wässrige beziehungsweise wässrigglyzerinische Auszüge aus Eibisch-Pflanzen (Althaea officinalis) als Arzneimittel, beispielsweise zur Behandlung von Entzündungen, bekannt. Da wiederum keine Fällung einer Fraktion erfolgt, gelangen wiederum alle Polysaccharide in die Auszüge, so dass ein grosser Teil der Polysaccharide in ihnen niedere Molekulargewichte unter 75 000 aufweist. Dies ergibt sich direkt aus Seite 2, linke Spalte, Zeilen 14 bis 15 der genannten Druckschrift, an welcher Stelle angegeben ist, dass deren Produkte die charakteristischen Reaktionen der $C_5$-Zucker zeigen (dies also nicht erst nach saurer Hydrolyse der Fall ist), welche aber Molekulargewichte um 150 haben. Daher ist die entzündungshemmende Wirkung dieser Produkte wiederum nur gering mit $ED_{50}$-Werten von etwa 1 500 bis 3 000. Überdies haben die einfachen wässrigen beziehungsweise wässrig-glyzerinischen Auszüge dieser Druckschrift je nach dem Jahr, dem Boden und dem vegetativen Zustand der Pflanze eine stark verschiedene Wirkung, weswegen sie für die praktische Arzneimittelzubereitung ohnehin nicht geeignet wären.

Weiterhin ist in der britischen Patentschrift 878 430 ein Polysaccharidmaterial, das von Pflanzen des Sassafras-Genus, das heisst einer bestimmten Lorbeerart, erhältlich ist, sowie ein Verfahren zu dessen Herstellung, bei welchem das Pflanzenmaterial zunächst mit Wasser extrahiert und der wässrige Auszug mit einem niederen aliphatischen Alkohol, wie Äthanol, behandelt wird, wobei nur der aus der Lösung mit einem Gehalt von 45 bis 75% eines der genannten Lösungsmittel enthaltenden Lösung erhaltene Niederschlag verwertet wird, während der aus der Lösung mit einem Gehalt an nur bis zu 45% des Lösungsmittels vorher gefällte Niederschlag verworfen wird, beschrieben. Damit wird ein Material, welches grösstenteils die Polysaccharide mit höheren Molekulargewichten enthält, verworfen, während das Material, welches grösstenteils die Polysaccharide mit niedrigem Molekulargewicht enthält, der pharmakologischen Verwendung zugeführt wird. So ist auf Seite 1, Zeilen 31 bis 39 der genannten Druckschrift angegeben, dass die Polysaccharide von deren Polysaccharidmaterial Molekulargewichte von 20 000 bis 400 000 aufweisen, wobei das durchschnittliche Molekulargewicht etwa 100 000 beträgt, und sie Galaktose, Arabinose, Rhamnose und Xylose enthalten. Die Angabe von Molekulargewichten von 20 000 bis 400 000 der Polysaccharide des Polysaccharidmateriales der genannten Druckschrift zeigt, dass dieses Polysaccharidmaterial Polysaccharide mit Molekulargewichten weit unter 50 000 enthält. Dies geht auch aus der Angabe des durchschnittlichen Molekulargewichtes von 100 000 hervor, da erfahrungsgemäss in Pflanzen das Molekulargewicht der Polysaccharide nach der Gaussschen Verteilungskurve verteilt ist, so dass es bei einem durchschnittlichen Molekulargewicht von 100 000 ausgeschlossen ist, dass kein Polysaccharid mit einem Molekulargewicht unter 75 000 vorliegt. Ferner ergibt sich dies aus der Angabe des Gehaltes von Galaktose, Arabinose, Rhamnose und Xylose ohne vorherige saure Hydrolyse, denn diese haben Molekulargewichte um 150. Als pharmakologische Wirkungen der Produkte der genannten Druckschrift sind nur die antivirale und antilipämische Wirksamkeit angegeben, während von einer entzündungshemmenden Wirkung keine Rede ist.

Ausserdem ist in «CHEMICAL & PHARMACEUTICAL BULLETIN», Band 25, Nr. 6, Juni 1977, Seiten 1 357 bis 1 362, das Isolieren und Charakterisieren von aus den Wurzeln von Althaea officinalis L gewonnenen Pflanzenschleimen, wobei zu ihrer Herstellung eine Extraktion mit kaltem Wasser und eine anschliessende Fällung mit Äthanol erfolgte, beschrieben. Als Molekulargewicht der Produkte dieser Druckschrift ist auf Seite 1 358, Zeile 14 von unten der niedrige Wert von nur 34 000 angegeben, wobei diese Produkte saure Polysaccharide sind. Von einer pharmakologischen Wirkung der Produkte der genannten Druckschrift ist in dieser keine Rede, lediglich für die Wurzeln der genannten Pflanze ist angegeben, dass sie eine erweichende und demulgente Wirkung sowie Hustenmedizinwirkung haben.

Schliesslich sind aus Chemical Abstracts, Band 91, Nr. 5, 30. Juli 1979, Seite 315, Nr. 35698q Polysaccharidhydrolysate aus der Droge Flos chamomillae mit einem Gehalt an Glukose, Fruktose, Arabinose, Xylose, Rhamnose und Galakturonsäure bekannt. Die Polysaccharide dieses Materiales haben aber ein niedriges Molekulargewicht von nur 4 350.

Der Erfindung liegt die Aufgabe zugrunde, vergleichbare pharmakologische, insbesondere entzündungshemmende, Wirkungen bei wesentlich geringerer Toxizität im Vergleich zu den üblichen Arzneimitteln und kosmetischen Mitteln aufweisende neue Polysaccharidkonzentrate pflanzlichen Ursprunges und diese Polysaccharidkonzentrate enthaltende Arzneimittel und kosmetische Präparate zu schaffen.

Das Obige wurde überraschenderweise durch die Erfindung erreicht.

Gegenstand der Erfindung sind Polysaccharidkonzentrate pflanzlichen Ursprungs mit

a) einem Molekulargewicht von 75 000 bis 2 000 000,

b) einem Gehalt an

α) höchstens 5 Gew.-% Stickstoff (Mikrokjeldahl-Verfahrensweise nach Wagner-Parnass; ungarische Norm 6830–66) und

β) höchstens 5 Gew.-% Phosphor, ausgedrückt als Phosphorpentoxyd, (mit Molybdänat-Eikonogen-Reagenz; Peac-Tracey: Moderne Methoden der Pflanzenanalyse, Band IV, Seite 308, Springer-Verlag, 1955, Berlin), die

c) höchstens 25 Gew.-% Glührückstand hinterlassen,

d)

α) mindestens 30 Gew.-% reduzierende Zucker (nach saurer Hydrolyse mit o-Toluidin bestimmt; R. Richterich: Klinische Chemie 1968, Seite 233, Carger Ed. Basel-New York) enthalten und

β) deren Gesamtzuckergehalt mindestens 60 Gew.-% (nach der Phenol-Schwefelsäure-Verfahrensweise bestimmt; M. Dubois: Anal. Chem. 28 [1956], Seite 35) beträgt, wobei

e) sie praktisch keine Stoffe mit Molekulargewichten unter 75 000 enthalten (untersucht mit einem Molekularsieb, bestehend aus einem dreidimensional vernetzten Polysaccharid, das durch Quervernetzung der linearen Makromoleküle von Dextran erhalten worden ist, mit etwa mittlerem Vernetzungsgrad,) [Sephadex G 75®] und

f) die nach saurer Hydrolyse mindestens 2 von den Bestandteilen Glucose, Galaktose, Xylose, Rhamnose, Arabinose und Uronsäure(n) enthalten, erhalten durch Behandeln von Polysaccharid enthaltenden Drogenpflanzen mit die Polysaccharide lösenden Mitteln, wobei man 1 oder mehr frische oder getrocknete, Polysaccharide enthaltende Drogenpflanzen, 1 bis 24 Stunden lang bei 0 bis 40 °C mit Wasser behandelt beziehungsweise extrahiert und danach oder dabei [jeweils] die Feststoffe entfernt und dann aus dem beziehungsweise den wässrigen Auszug beziehungsweise Auszügen {flüssigen Teil(en)} durch Zugabe von 1 oder mehr Alkohol(en) mit 1 bis 3 Kohlenstoffatom(en), Aceton und/oder 1- bis 3-wertigen Kationen des Polysaccharidkonzentrat fällt und dieses von der wässrigen Phase abtrennt, welche dadurch gekennzeichnet sind, dass sie gewonnen werden durch Aufarbeitung von 1 oder mehr Pflanzen der folgenden Pflanzenfamilien: Kürbis (Cucurbitaceae), Schmetterlingsblütler (Papilionaceae), lindenartige Gewächse (Tiliaceae), Lippenblütler (Labiatae), Wurzeln der weissen Malve (Althaeae radix), Malvenblatt oder -blüte (Malvae folium et flos), Kraut der Färbermalve (Malvae arboreae herba), Korbblütler (Compositae beziehungsweise Asteraceae), Doldenblütler (Umbellifereae), Rautengewächse (Rutaceae), Gänsefussgewächse (Chenopodiaceae), leinartige Gewächse (Linaceae), Rosengewächse (Rosaceae) und wegerichartige Gewächse (Plantaginaceae), wobei diese Polysaccharidkonzentrate

g) im Carraghenin-Ödem-Versuch an Rattenpfoten eine mit der Wirkung von 4-Butyl-1,2-di-(phenyl)-pyrazolidin-3,5-dion {Phenylbutazon} und 1-[4'-(Chlor)-benzoyl]-5-[methoxy]-2-[3''-(methyl)-indolyl]-essigsäure {Indometacin} vergleichbare entzündungshemmende Wirkung zeigen und

h) ihr peroral bestimmter $LD_{50}$-Wert an Mäusen grösser als 3 000 mg/kg Körpergewicht und an Ratten grösser als 2 000 mg/kg Körpergewicht ist.

Bevorzugt sind die erfindungsgemässen Polysaccharidkonzentrate solche, welche durch Aufarbeitung von 1 oder mehr der folgenden Pflanzen beziehungsweise Pflanzenteile gewonnen worden sind: Kamillenblüte (Chamomillae flos), Lindenblüte (Tiliae flos), Wegerichblatt (Plantaginis folium), Flohkrautsamen (Psyllii semen) [Samen von Pulicaria], Quittensamen beziehungsweise -kern (Cydoniae semen), Leinsaat (Lini semen), Bockshornkleesamen (Trigonella foeni-graeci semen), Kürbis (Cucurbitae maximae fructus) und Kitaibela vitifolia herba.

Nach einer besonders bevorzugten Ausführungsform der erfindungsgemässen Polysaccharidkonzentrate sind sie aus Kürbis (Cucurbita maxima) hergestellt, ihr Molekulargewicht ist grösser als 300 000, sie enthalten höchstens 3 Gew.-% Stickstoff, sie hinterlassen höchstens 20 Gew.-% Glührückstand und sie enthalten nach saurer Hydrolyse vor allem Glucose, Galaktose und Uronsäuren und in einer Dosis von 10 mg/kg Körpergewicht beträgt ihre entzündungshemmende Wirkung im Carraghenin-Ödem-Versuch an Rattenpfoten mindestens 50%.

Nach einer weiteren besonders bevorzugten Ausführungsform der erfindungsgemässen Polysaccharidkonzentrate sind sie aus Bockshornkleesamen (Trigonella foeni-graeci semen) hergestellt, ihr Molekulargewicht ist grösser als 100 000, sie enthalten höchstens 3 Gew.-% Stickstoff und höchstens 4 Gew.-% Phosphor, ausgedrückt als Phosphorpentoxyd, sie hinterlassen höchstens 5 Gew.-% Glührückstand, sie enthalten mindestens 50 Gew.-% reduzierende Zucker und mindestens 70 Gew.-% Gesamtzucker und sie enthalten nach saurer Hydrolyse vor allem Galaktose und Mannose und in einer Dosis von 10 mg/kg Körpergewicht beträgt ihre entzündungshemmende Wirkung im Carraghenin-Ödem-Versuch an Rattenpfoten mindestens 40%.

Ferner wurde überraschenderweise festgestellt, dass aus Drogenpflanzen, insbesondere den oben aufgezählten, und zwar sowohl aus den frischen als auch aus den getrockneten, in von den üblichen Extraktionsverfahren abweichender Weise, nämlich bei der Umgebungstemperatur oder wenig darunter oder darüber mit Wasser, vorzugsweise in geringer Menge, eine Polysaccharidfraktion, die eine bedeutende entzündungshemmende Wirkung hat, isoliert werden kann. Diese Polysaccharidfraktion kann gegebenenfalls durch Umfällen und andere Reinigungsverfahren gereinigt werden.

Vorzugsweise werden die erfindungsgemässen Polysaccharidkonzentrate gewonnen durch Behandeln von Polysaccharide enthaltenden Drogenpflanzen mit die ersteren lösenden Mitteln, wobei 1 oder mehr frische oder getrocknete Polysaccharide enthaltende Drogenpflanze(n) 1 bis 24 Stunden lang bei 0 bis 40 °C mit Wasser behandelt beziehungsweise extrahiert wird beziehungsweise werden und danach oder dabei [jeweils] die Feststoffe entfernt werden und dann aus dem beziehungsweise den wässrigen Auszug beziehungsweise Auszügen {flüssigen Teil(en)} durch Zugabe von 1 oder mehr Alkohol(en) mit 1 bis 3 Kohlenstoffatom(en), Aceton und/oder 1- bis 3-wertigen Kationen das Polysaccharidkonzentrat gefällt und dieses von der wässrigen Phase abgetrennt wird, mit der weiteren Massgabe, dass zum Fällen des Polysaccharidkonzentrates der beziehungsweise die Alkohol(e) im 1- bis 7-fachen Volumen und das Aceton im bis zu 10-fachen Volumen, jeweils bezogen auf den wässrigen Auszug, und die 1- bis 3-wertigen Kationen in der $^1/_{20}$-sten bis $^1/_5$-ten Menge, bezogen auf das Gewicht der Drogenpflanzen, verwendet wird beziehungsweise werden.

Nach einer bevorzugten Ausführungsform werden als Polysaccharide enthaltende Drogenpflanzen solche, welche mit 1 oder mehr organischen Lösungsmittel(n) vorbehandelt worden sind, verwendet. Dabei ist es bevorzugt, als Polysaccharide enthaltende Drogenpflanzen solche, zu deren Vorbehandlung als organische[s] Lösungsmittel [ein] wässrige[s] eingesetzt worden ist beziehungsweise sind, zu verwenden. Ferner ist es bevorzugt, als Polysaccharide enthaltende Drogenpflanzen solche, deren gegebenenfalls erfolgende Vorbehandlung mit dem beziehungsweise den organischen Lösungsmittel(n) nach einem Desintegrieren durchgeführt worden ist, zu verwenden.

Nach einer zweckmässigen Ausführungsform wird die Behandlung bzw. das Extrahieren mit dem Wasser mehrmals durchgeführt.

Nach einer weiteren vorteilhaften Ausführungsform wird bzw. werden die Behandlung(en) beziehungsweise das beziehungsweise die Extraktion(en) mit dem Wasser nach einem oder während eines Desintegrieren[s] durchgeführt.

Vorzugsweise wird beziehungsweise werden zur gegebenenfalls erfolgenden Vorbehandlung der Drogenpflanzen als organische[s] Lösungsmittel 1 oder mehr, gegebenenfalls wässrige[r], Alkohol(e), insbesondere Methanol und/oder Äthanol, verwendet. Vorteilhaft wird diese Vorbehandlung 1 bis 24 Stunden, insbesondere 24 Stunden, durchgeführt. Danach kann der Drogenpflanzenfeststoff durch Pressen und Filtrieren abgetrennt und gegebenenfalls, zweckmässig bei Raumtemperatur, getrocknet werden.

Vorteilhaft wird zur beziehungsweise zu den Behandlung(en) mit Wasser die 1- bis 20-fache Wassermenge, bezogen auf das Gewicht der Drogenpflanze, verwendet.

Zweckmässig wird das gegebenenfalls erfolgende Desintegrieren der Drogenpflanzen durch Zerkleinern und/oder Mahlen durchgeführt. Es ist bevorzugt, im wässrigen Medium ein Desintegrieren durchzuführen. Getrocknete Drogen können trocken oder nass zerkleinert werden. Die Behandlung beziehungsweise Extraktion mit Wasser kann aber auch ohne Desintegrieren vorgenommen werden, zum Beispiel im Falle von Samen oder grobzerkleinerten Wurzelstücken.

In vielen Fällen ist es zweckmässig, vor und/oder nach der beziehungsweise den Behandlung(en) mit Wasser die Drogenpflanzen auszupressen und die wässrige(n) Phase(n) weiterzubehandeln. Im Falle des Vorliegens von mehreren Fraktionen von wässrigen Phasen ist es bevorzugt, diese vor ihrem Weiterbehandeln zu vereinigen. Nach einer zweckmässigen Ausführungsform des erfindungsgemässen Verfahrens wird beziehungsweise werden daher die Behandlung(en) mit dem Wasser und das Abtrennen der Feststoffe in der Weise durchgeführt, dass nach dem Desintegrieren der, gegebenenfalls gewässerten, frischen Drogenpflanze(n) oder der, gegebenenfalls gewässerten, getrockneten Drogenpflanze(n) und Auspressen des erhaltenen Breies der Pressrückstand [erneut] mit Wasser behandelt und erneut ausgepresst wird. Aus den vereinigten wässrigen Phasen kann dann das Entfernen der Feststoffe erfolgen.

In einigen Fällen ist es vorteilhaft, die Temperatur bei der beziehungsweise den Behandlung(en) mit Wasser durch Kühlen, beispielsweise auf etwa 4 °C, niedrig zu halten, für bestimmte Fälle haben sich jedoch andererseits Temperaturen um + 30 °C als vorteilhaft erwiesen.

Im erfindungsgemässen Verfahren wird beziehungsweise werden als Alkohol(e) vorzugsweise Methanol und/oder Äthanol verwendet. Dies gilt für alle Fällungen und Wascharbeitsgänge.

Als 1-wertige Kationen werden vorteilhaft Alkaliionen, insbesondere Ammoniumionen, die beispielsweise mit Hilfe von Ammoniumsulfat eingeführt werden können, verwendet.

Als 2-wertige Kationen werden vorteilhaft Erdalkalimetallionen, insbesondere Bariumionen, die beispielsweise mit Hilfe von Bariumhydroxyd eingeführt werden können, verwendet.

Das kontinuierliche Aufarbeiten ist bevorzugt. Daher wird vorzugsweise das Ausgangsmaterial nass zerkleinert und unter niedrigem oder hohem Druck mehrmals ausgepresst und gegebenenfalls die gepresste Masse erneut mit Wasser behandelt. Die ausgepresste beziehungsweise extrahierte und durch Pressen abgetrennte Flüssigkeit enthält die Polysaccharidfraktion. Aus diesem wässrigen Medium wird dann das Fällen dieser Polysaccharidfraktion mit 1 oder mehr der angegebenen Fällungsmittel vorgenommen.

Besonders bevorzugt wird das Entfernen der im wässrigen Auszug befindlichen eiweissartigen Stoffe in der Weise durchgeführt, dass der wässrige Auszug auf 40 bis 80 °C, insbesondere 60 °C, erwärmt und der ausgefallene Niederschlag abgetrennt wird.

Nach einer vorteilhaften Ausführungsform des erfindungsgemässen Verfahrens wird das Fällen des Polysaccharidkonzentrates mit dem beziehungsweise den Alkohol(en), Aceton und/oder 1- bis 3-wertigen Kationen nach Abtrennen der eiweissartigen Stoffe und/oder Einengen auf ein geringes Volumen durchgeführt.

Vorzugsweise wird das gegebenenfalls erfolgende Abtrennen des Eiweissniederschlages durch Zentrifugieren und/oder Filtrieren durchgeführt.

Es ist auch bevorzugt, zum Fällen des Polysaccharidkonzentrates den beziehungsweise die Alkohol(e) im 1- bis 4-fachen, ganz besonders 1- bis 3-fachen, vor allem 2- bis 3-fachen, Volumen, bezogen auf den wässrigen Ausug, zu verwenden.

Ferner ist es bevorzugt, so vorzugehen, dass das Abtrennen des Polysaccharidkonzentrates von der wässrigen Phase nach einem, insbesondere 1 bis 36 Stunden langen, ganz besonders 24 Stunden langen, Stehenlassen durch Zentrifugieren und/oder Filtern durchgeführt wird, worauf es mit dem Fällungsmittel, insbesondere Alkohol, gegebenenfalls mehrmals, gewaschen wird.

Das Gewicht des gewinnbaren Polysaccharidkonzentrates beträgt 0,1 bis 10 Gew.-%, bezogen auf den Trockengehalt des Ausgangsmateriales.

Vorzugsweise wird das erhaltene Polysaccharidkonzentrat gereinigt.

Vorteilhaft wird das Reinigen des Polysaccharidkonzentrates durch, gegebenenfalls mehrfaches, Umfällen durchgeführt.

Vorzugsweise wird das Reinigen durch Umfällen des 1-mal gefällten rohen Polysaccharidkonzentrates in der Weise durchgeführt, dass es in Wasser gelöst und mit einem zum ersten Fällen verwendbaren Fällungsmittel, insbesondere Alkohol, gefällt wird, wobei die Reihenfolge der Zugabe des Wassers und Fällungsmittels beliebig gewählt werden kann, und gegebenenfalls das Obige 1-mal oder mehrmals wiederholt wird.

Besonders bevorzugt wird das Reinigen durch Umfällen des 1-mal gefällten rohen Polysaccharidkonzentrates in der Weise durchgeführt, dass es in der 5- bis 15-fachen, insbesondere 10-fachen, Menge Wasser, bezogen auf sein Gewicht, gelöst und auf 65 bis 80 °C, insbesondere 75 °C, erwärmt wird, die ausgefallenen Substanzen durch Zentrifugieren entfernt werden, aus der klaren Lösung das Polysaccharidkonzentrat durch Zugabe eines zum ersten Fällen verwendbaren Fällungsmittels, insbesondere Alkoholes, gefällt wird, das Gemisch, insbesondere 1 bis 24 Stunden lang, ganz besonders 24 Stunden lang, stehengelassen wird und dann der Niederschlag durch Zentrifugieren und/oder Filtrieren abgetrennt, mit dem Fällungsmittel, insbesondere Alkohol, gewaschen und unter Vakuum bei höchstens 60 °C, insbesondere höchstens 40 °C, getrocknet wird sowie gegebenenfalls das Obige 1-mal oder mehrmals wiederholt wird. Es handelt sich also um das Fällen des Polysaccharidkonzentrates aus der wässrigen Lösung durch Zugabe des Fällungsmittels, vorzugsweise Alkoholes, Lösen des Polysaccharidkonzentratniederschlages in Wasser und erneutes Fällen des Polysaccharidkonzentrates mit dem Fällungsmittel und auf diese Weise dessen Reinigen durch wiederholtes Lösen und/oder Extrahieren und Fällen mit dem Fällungsmittel.

Die oben für das erste Fällen angegebenen beziehungsweise bevorzugten Mengen des Fällungsmittels sind beim Fällen bei dieser Reinigung bevorzugt beziehungsweise besonders bevorzugt. So wird beziehungsweise werden der beziehungsweise die Alkohol(e) vorzugsweise in der 1- bis 7-fachen, insbesondere 1- bis 4-fachen, ganz besonders 1- bis 3-fachen, vor allem 2- bis 3-fachen, Menge, bezogen auf die klare Lösung des Polysaccharidkonzentrates, verwendet.

Das bei dieser Reinigung gegebenenfalls erfolgende Eindampfen wird zweckmässig unter vermindertem Druck bei Temperaturen von höchstens 60 °C durchgeführt. Ein etwaiger ausgeschiedener Niederschlag wird entfernt.

Bei dieser Reinigung können die einzelnen Fällungsverfahrensweisen auch kombiniert werden, wobei dies auch für die Verwendung von verschiedenen Fällungsmitteln im Falle von mehrmaligem Fällen gilt.

Als bevorzugte andere oder zusätzliche Möglichkeit wird das Reinigen beziehungsweise Weiterreinigen des erhaltenen rohen beziehungsweise gereinigten Polysaccharidkonzentrates in der Weise durchgeführt, dass es in der 25- bis 40-fachen, insbesondere 30 bis 40-fachen, Menge Wasser, bezogen auf sein Gewicht, bei 50 bis 75 °C gelöst wird, die Lösung durch Zentrifugieren gereinigt und dann unter Überdruck, insbesondere unter einem Druck von 0,3 bis 1,0 MPaü, durch ein die Stoffe mit einem Molekulargewicht unter 75 000 durchlassendes Membranfilter gedrückt wird und aus der auf der hochmolekularen Seite des Membranfilters gebliebenen Lösung, gegebenenfalls nach ihrem Verdünnen mit Wasser, mit einem zum ersten Fällen verwendbaren Fällungsmittel, insbesondere Alkohol, das Polysaccharid gefällt wird, wobei gegebenenfalls das Lösen in Wasser und Reinigen durch Zentrifugieren und/oder Membranfiltrieren und/oder Fällen 1-mal oder mehrmals wiederholt wird beziehungsweise werden. Zwar wird auch dabei als Fällungsmittel bevorzugt ein Alkohol verwendet, es können aber auch die anderen genannten Fällungsmittel, wie Aceton, eingesetzt werden.

Die oben für das erste Fällen angegebenen beziehungsweise bevorzugten Mengen des Fällungsmittels sind beim Fällen bei dieser Reinigung bevorzugt beziehungsweise besonders bevorzugt. So wird beziehungsweise werden der beziehungsweise die Alkohol(e) vorzugsweise in der 1- bis 7-fachen, insbesondere 1- bis 4-fachen, ganz besonders 1- bis 3-fachen, vor allem 2- bis 3-fachen, Menge, bezogen auf die klare Lösung des Polysaccharidkonzentrates, verwendet.

Ferner sind erfindungsgemäss Arzneimittel, welche 1 oder mehr der erfindungsgemässen Polysaccharidkonzentrate als Wirkstoffe, gegebenenfalls zusammen mit 1 oder mehr anderen nicht-steroiden entzündungshemmenden Stoff(en), insbesondere 1-[4'-(Chlor)-benzoyl]-5-[methoxy]-2-[3''-(methyl)-indolyl]-essigsäure {Indometacin} und/oder Acetylsalicylsäure und/oder 1 oder mehr Derivat(en) derselben, und/oder 1 oder mehr üblichen pharmazeutischen Träger- und/oder Hilfsstoff(en), enthalten, vorgesehen.

Weiterhin sind erfindungsgemäss kosmetische Präparate, welche 1 oder mehr der erfindungsgemässsen Polysaccharidkonzentrate als Wirkstoffe, gegebenenfalls zusammen mit 1 oder mehr üblichen äusserlich anwendbaren kosmetischen Träger- und/oder Hilfsstoff(en), enthalten, vorgesehen.

Die erfindungsgemässen Polysaccharidkonzentrate haben nämlich wie bereits erwähnt wertvolle pharmakologische, insbesondere entzündungshemmende, Wirkungen.

Die entzündungshemmende Wirkung der erfindungsgemässen Polysaccharidkonzentrate wurde bei allen Untersuchungen nach der Verfahrensweise von C.A. Winter, E.A. Risley und G.W. Nuss (Proc. Soc. Exp. Biol. Med. 111 [1962], 544) im Carraghenin-Ödem-Versuch an Rattenpfoten untersucht. Sie zeigten eine bedeutende spezifische Aktivität und eine damit zusammenhängende ausserordentlich günstige entzündungshemmende Wirkung bei auffallend niedriger Toxizität. Auf Grund der niedrigeren Toxizität der erfindungsgemässen Polysaccharidkonzentrate noch dazu vielfach zusammen mit einer höheren entzündungshemmenden Wirkung derselben als bei den Vergleichssubstanzen sind die therapeutischen Indices der ersteren höher als die von allen untersuchten Vergleichssubstanzen.

Speziell wurden die folgenden pharmakologischen Untersuchungen durchgeführt.

Zunächst wurde die entzündungshemmende Wirkung der erfindungsgemässen Polysaccharidkonzentrate im Carraghenin-Ödem-Versuch an Rattenpfoten nach intraperitonealer Verabreichung untersucht, wobei als Vergleichssubstanz 4-Butyl-1,2-di-(phenyl)-pyrazolidin-3,5-dion [Phenylbutazon] {Vergleichssubstanz A} diente. Gleichzeitig wurde auch die Toxizität als intraperitonealer $LD_{50}$-Wert bestimmt. Die Ergebnisse sind in der folgenden Tabelle 1 zusammengestellt.

Tabelle 1

| Polysaccharidkonzentrat | | Dosis in mg/kg | Hemmung in % | $LD_{50}$-Wert in mg/kg | Dem therapeutischen Index proportionaler Wert $LD_{50}$-Wert × Hemmung |
|---|---|---|---|---|---|
| Beispiel | Ausgangsmaterial | | | | |
| 1 | frische Kamillenblüten [Chamomillae flos] | 50 | 74,1 | 1 500 | 111 150 |
| 2 | getrocknete Kamillenblüten [Chamomillae flos] | 100 | 71,7 | 1 500 | 107 550 |
| 17 | getrocknete Lindenblüten [Tiliae flos] | 100 | 80,3 | 1 000 | 80 300 |
| 10 | frische Wurzeln der weißen Malve [Althaeae radix] | 100 | 54,2 | 1 500 | 78 750 |
| 8 | frische Quittenkerne [Cydoniae semen] | 100 | 52,5 | 1 000 | 52 500 |
| 9 | getrocknete Leinsaat [Lini semen] | 100 | 43,8 | 1 000 | 43 800 |
| Vergleichs- substanz A | 4-Butyl-1,2-di-(phenyl)- pyrazolidin-3,5 dion [Phenylbutazon] | 100 | 45,0 | 215 | 9 675 |

Aus der obigen Tabelle 1 geht eindeutig die Überlegenheit der erfindungsgemässen Polysaccharidkonzentrate gegenüber der Vergleichssubstanz 4-Butyl-1,2-di-(phenyl)-pyrazolidin-3,5-dion [Phenylbutazon] {Vergleichssubstanz A} hervor, da die dem therapeutischen Index proportionalen Werte bei den ersteren 4,5- bis 11,5-mal so gross wie bei der letzteren waren.

Die entzündungshemmende Wirkung von erfindungsgemässen Polysaccharid-Konzentraten wurde experimentell auch mit der 1-[4'-(chlor)-benzoyl]-5-[methoxy]-2-[3''-(methyl)-indolyl]-essigsäure [des Indometacines] {Vergleichssubstanz B} verglichen. Es wurde derselbe Carraghenin-Ödem-Versuch wie oben durchgeführt. Die zu untersuchenden Polysaccharidkonzentrate wurden 1 Stunde vor der Verabreichung des die Entzündung auslösenden Carraghenines interperitonal injiziert. Die Vergleichssubstanz 1-[4'-(Chlor)-benzoyl]-5-[methoxyl]-2-[3''-(methyl)-indolyl]-essigsäure [Indometacin] {Vergleichssubstanz B} wurde peroral verabreicht, und zwar CFY-Ratten 1 Stunde vor dem Carraghenin in Suspension in 1%-iger Methylcellulose durch eine Magensonde. Auch bei den Toxizitätsuntersuchungen wurde ähnlich vorgegangen. Die entzündungshemmende Wirkung als $ED_{50}$-Werte, die Toxizität als $LD_{50}$-Werte und die therapeutischen Indices von erfindungsgemässen Polysaccharidkonzentraten und der Vergleichssubstanz 1-[4'-(Chlor)-benzoyl]-5-[methoxy]-2-[3''-(methyl)-indolyl]-essigsäure [Indometacin] {Vergleichssubstanz B} sind in der folgenden Tabelle 2 zusammengestellt (soweit nichts anderes angegeben ist, wurde an Ratten intraperitoneal verabreicht, wobei bemerkt sei, dass die erfindungsgemässen Polysaccharidkonzentrate eine so geringe Toxizität haben, dass zur peroralen Toxizitätsbestimmung zu grosse Dosen verabreicht werden mussten, was bei kleinen Tieren schwierig ist).

Tabelle 2

| Polysaccharidkonzentrat | | Entzündungshemmende Wirkung als $ED_{50}$-Wert in mg/kg nach | | $LD_{50}$-Wert in mg/kg | Therapeutischer Index $\dfrac{LD_{50}\text{-Wert}}{ED_{50}\text{-Wert}}$ nach | |
|---|---|---|---|---|---|---|
| Beispiel | Ausgangsmaterial | 2 Stunden | 5 Stunden | | 2 Stunden | 5 Stunden |
| 19 | Kürbis [Cucurbita maxima] | 0,51 | 0,37 | 112 | 219,6 | 302,7 |
| 7 | getrocknete Lindenblüten [Tiliae flos] | 2,83 | 2,20 | 150 | 53,0 | 68,2 |
| 20 | Bockshornkleesamen [Trigonella foeni graeci semen] | 2,19 | 1,17 | 200 | 91,3 | 170,9 |
| Vergleichssubstanz B | 1-[4'-(Chlor)-benzoyl]-5-[methoxy]-2-[3''-(methyl)-indolyl]-essigsäure [Indometacin] {peroral verabreicht} | 3,14 | 2,20 | 32 | 10,2 | 14,5 |

In der folgenden Tabelle 3 ist die Wirkungsstärke der obigen erfindungsgemässen Polysaccharidkonzentrate mit der der 1-[4'-(Chlor)-benzoyl]-5-[methoxy]-2-[3''-(methyl)-indolyl]-essigsäure

[des Indometacines] {Vergleichssubstanz B} verglichen. Dabei ist die relative Wirkungsstärke, das heisst der Quotient aus dem $ED_{50}$-Wert des letzteren und dem $ED_{50}$-Wert des jeweiligen Polysaccharidkonzentrates, angegeben.

Tabelle 3

| Polysaccharidkonzentrat | | Relative Wirkungsstärke $\dfrac{ED_{50}\text{-Wert der 1-[4'-(Chlor)-benzoyl]-5-[methoxy]-2-[3''-(methyl)-indolyl]-essigsäure}}{ED_{50}\text{-Wert des Polysaccharidkonzentrates nach}}$ | |
|---|---|---|---|
| Beispiel | Ausgangsmaterial | 2 Stunden | 5 Stunden |
| 19 | Kürbis [Cucurbita maxima] | 6,16 | 5,95 |
| 7 | getrocknete Lindenblüten [Tiliae flos] | 1,11 | 1,00 |
| 20 | Bockshornkleesamen [Trigonella foeni-graeci semen] | 1,43 | 1,88 |

Aus den obigen Tabellen 2 und 3 geht die Überlegenheit der erfindungsgemässen Polysaccharidkonzentrate gegenüber der Vergleichssubstanz 1-[4'-(Chlor)-benzoyl]-5-[methoxy]-2-[3''-(methyl)-indolyl]-essigsäure [Indometacin] {Vergleichssubstanz B} hervor, wobei die therapeutischen Indices der ersteren 4,7- bis 20,9-mal so hoch wie die der letzteren waren.

In der folgenden Tabelle 4 sind pharmazeutische Daten von erfindungsgemässen Polysaccharidkonzentraten mit denen von verbreitet eingesetzten bekannten entzündungshemmenden Substanzen verglichen, wobei die Daten für die erfindungsgemässen Polysaccharidpräparate nach der für die Tabelle 2 die dort stehenden erfindungsgemässen Polysaccharidpräparate betreffend angegebenen Verfahrensweise und für die Vergleichssubstanzen
1-[4'-(Chlor)-benzoyl]-5-[methoxy]-2-[3''-(methyl)-indolyl]-essigsäure [Indometacin] {Vergleichssubstanz B},
4-Butyl-1,2-di-(phenyl)-pyrazolidin-3,5-dion

[Phenylbutazon] {Vergleichssubstanz A} und Acetylsalicylsäure [Aspirin] {Vergleichssubstanz C} nach der für die Tabelle 2 die dort stehende Vergleichssubstanz betreffend angegebenen Verfahrensweise erhalten wurden [soweit nichts anderes angegeben ist, wurde an Ratten intraperitoneal verabreicht].

Tabelle 4

| Polysaccharidkonzentrat | | Dosis in mg/kg | Entzündungs- hemmende Wirkung in % | $LD_{50}$-Wert in mg/kg | $\dfrac{Dosis}{LD_{50}\text{-Wert}}$ |
|---|---|---|---|---|---|
| Beispiel | Ausgangsmaterial | | | | |
| 19 | Kürbis [Cucurbita maxima] | 10 | 54 | 214 | 0,047 |
| 20 | Bockshornkleesamen [Trigonella foeni-graeci semen] | 10 | 40 | 250 | 0,040 |
| Vergleichs- substanz B | 1-[4'-(Chlor)-benzoyl]-5-[methoxy]-2-[3''-(methyl)-indolyl]-essigsäure [Indometacin] {peroral verabreicht} | 10 | 32 | 17 | 0,313 |
| Vergleichs- substanz A | 4-Butyl-1,2-di-(phenyl)-pyrazolidin-3,5-dion [Phenylbutazon] {peroral verabreicht} | 90 | 40 | 470 | 0,191 |
| Vergleichs- substanz C | Acetylsalicylsäure [Aspirin] {peroral verabreicht} | 400 | 36 | 1 200 | 0,333 |

Aus der obigen Tabelle 4 geht wiederum die Überlegenheit der erfindungsgemässen Polysaccharidkonzentrate gegenüber den Vergleichssubstanzen
1-[4'-(Chlor)-benzoyl]-5-[methoxy]-2-[3''-(methyl)-indolyl]-essigsäure [Indometacin] {Vergleichssubstanz B},
4-Butyl-1,2-di-(phenyl)-pyrazolidin-3,5-dion
[Phenylbutazon] {Vergleichssubstanz A} und Acetylsalicylsäure [Aspirin] {Vergleichssubstanz C} hervor, wobei die Werte von

$$\frac{Dosis}{LD_{50}\text{-Wert}}$$

bei den erfindungsgemässen Polysaccharidkonzentraten 4- bis 4,8-mal geringer als bei der besten Vergleichssubstanz, dem
4-Butyl-1,2-di-(phenyl)-pyrazolidin-3,5-dion
[Phenylbutazon] {Vergleichssubstanz A} waren.
Die pharmakologischen Untersuchungen erbrachten ferner den Beweis, dass die erfindungsgemässen Polysaccharidkonzentrate beziehungsweise ihr entzündungshemmender Wirkstoff durch Beeinflussung der Chininphase der Entzündung wirken. Wie es bekannt ist, werden in der Entwicklung des Carraghenin-Ödemes 3 Phasen unterschieden, nämlich die Aminphase (Histamin, Serotonin), die Chininphase (Bradykinin) und die Prostaglandinphase (PG). Zur Herausbildung der Prostaglandinphase sind vom Einspritzen des die Entzündung auslösenden Carraghenines an gerechnet 5 Stunden erforderlich. Die erfindungsgemässen Polysaccharidkonzentrate hemmen die Synthese des Prostaglandines nicht, da sie keine geschwürbildende beziehungsweise ulcerogene Wirkung haben. Dies ist deshalb besonders hervorzuheben, weil die nicht-steroiden entzündungshemmenden Mittel und die antirheumatischen Mittel, die ihre entzündungshemmende Wirkung durch die Hemmung des Enzymes Prostaglandin-Synthetase ausüben, im Magen und im Darm Geschwüre verursachen. Durch Kombination der nicht-steroiden entzündungshemmenden Mittel, in erster Linie der
1-[4'-(Chlor)-benzoyl]-5-[methoxy]-2-[3''-(methyl)-indolyl]-essigsäure
[des Indometacines]
und der Acetylsalicylsäurederivate, mit den erfindungsgemässen Polysaccharidkonzentraten kann die geschwürbildende Wirkung dieser Wirkstoffe beseitigt werden. Die toxikologischen Untersuchungen zeigten, dass für die erfindungsgemässen Polysaccharidkonzentrate an Ratten und Mäusen eine ausserordentlich steile Dosis/Wirkungs-Kurve charakteristisch ist. Die intraperitoneale Verabreichung von $1/3$ beziehungsweise $1/10$ der akuten $LD_{50}$-Werte über 8 Tage führte praktisch weder bei Ratten noch bei Mäusen zum Tode.
Die biologische Wirkung der erfindungsgemässen Polysaccharidkonzentrate konnte einfach dadurch veranschaulicht werden, dass aus den erfindungsgemässen Polysaccharidkonzentraten eine Lösung der Konzentration von 0,5 Gew.-% in Wasser oder einer physiologischen Kochsalzlösung, gegebenenfalls mit Zusätzen von Konservierungsmitteln, hergestellt wurde. Diese Lösung ist zum Spülen der Mund- und Rachenhöhle bei Rachenkatarrh [Pharyngitis] und Zahnfleischentzündung [Gingivitis], zum Spülen der Augen bei Bindehautentzündung [Conjunctivitis], zum Spülen der Ohren beziehungsweise zum Einführen eines mit ihr getränkten Gazestreifens als Kompresse in die letzteren bei entzündlichen Erkrankungen der äusseren Gehörwege geeignet. Mit den erfindungsgemässen Polysaccharidkonzentraten bereitete Sitzbäder haben sich zur Behandlung von Fissura ani, Nodus hemorrhoidales Scheidenkatarrh [Vaginitis] und Vulvovaginitis bewährt und mit den erfindungsgemässen Polysaccharidkonzentraten getränkte Umschläge sind bei Rissen der Brustwarze [Rhagad] und bei Hautentzündung [Dermatitis] angebracht.

Die erfindungsgemässen Polysaccharidkonzentrate können in Form von Arzneimittelpräparaten vorliegen. Diese und die erfindungsgemässen kosmetischen Präparate können nach an sich bekannten Verfahrensweisen durch Vermischen von 1 oder mehr erfindungsgemässen Polysaccharidkonzentrat(en) und gegebenenfalls anderen nichtsteroiden entzündungshemmenden Stoff(en) mit 1 oder mehr üblichen pharmazeutischen Träger- und/oder Hilfsstoff(en) beziehungsweise äusserlich anwendbaren kosmetischen Träger und/oder Hilfsstoff(en) hergestellt werden.

Die erfindungsgemässen Arzneimittelpräparate können vorteilhaft in peroral oder parenteral verabreichbarer Form, wie in Form von Tabletten, Dragees, Kapseln, Emulsionen, Lösungen oder Suspensionen, beispielsweise Injektionslösungen oder -suspensionen oder Infusionslösungen oder äusserlich anzuwendenden Lösungen, Transplantaten, Zäpfchen, Cremes, Salben oder Gelen, vorliegen.

Die erfindungsgemässen kosmetischen Präparate können vorteilhaft als Lösungen, Emulsionen, Cremes, Salben, Gele, Schäume, Seifen, Zahncremes, Milch oder Körperpuder vorliegen.

Zu den aus «CHEMICAL & PHARMACEUTICAL BULLETIN», Band 25, Nr. 6, Juni 1977, Seiten 1 357 bis 1 362 bekannten Produkten haben die erfindungsgemässen Polysaccharidkonzentrate den grundlegenden Unterschied, dass sie viel höhere Molekulargewichte aufweisen und praktisch frei von Stoffen mit Molekulargewichten unter 75 000 sind. Analoges gilt gegenüber den aus «CHEMICAL ABSTRACTS», Band 91, Nr. 5, 30. Juli 1979, Seite 315, Nr. 35 698q beschriebenen Materialien.

Die Erfindung wird an Hand der folgenden Beispiele näher erläutert.

Beispiel 1

200 g frische Kamillenblüten werden unter gleichzeitiger wässriger Desintegrierung mit 400 ml Wasser homogenisiert. Das Gemisch wird bei 20 °C 20 Stunden lang stehen gelassen. Der wässrige Teil wird durch Zentrifugieren abgetrennt, in der Flüssigkeit verbliebene Feststoffe werden durch Filtrieren entfernt. Unter Rühren werden 1000 ml Methanol zu dem wässrigen Auszug gegeben. Die ausgefallene Substanz wird abfiltriert, mit Methanol gewaschen und dann getrocknet. 0,3 g Polysaccharid werden erhalten. Werden zum Ausfällen statt des Methanols 1000 ml Äthanol verwendet, so erhält man 0,32 g Konzentrat.

Beispiel 2

200 g getrocknete Kamillenblüten werden mit 1000 ml Wasser nass desintegriert. Dann wird das Gemisch bei 20 °C 2 Stunden lang stehen gelassen. Der wässrige Teil wird durch Zentrifugieren und Filtrieren gereinigt und dann mit 2500 ml Methanol versetzt. Der ausgefallene Niederschlag wird abfiltriert und mit Methanol gewaschen. 2,12 g Polysaccharid-Konzentrat werden erhalten.

Beispiel 3

200 g frische Kamillenblüten werden gemahlen, dann wird das Mahlgut ausgepresst. Der Pressrückstand wird bei 20 °C eine Stunde lang in 200 ml Wasser eingeweicht und dann erneut ausgepresst. Die vereinigten wässrigen Phasen werden filtriert und dann unter Rühren mit 600 ml Methanol versetzt. Der ausgefallene Niederschlag wird abfiltriert, mit Methanol gewaschen und dann getrocknet. 0,26 g Polysaccharid-Konzentrat werden erhalten.

Beispiel 4

Man arbeitet auf die im Beispiel 3 beschriebene Weise mit dem Unterschied, dass man den wässrigen Auszug mit 2000 ml Aceton als Fällungsmittel versetzt. Der abfiltrierte Niederschlag wird mit Aceton gewaschen und dann getrocknet. 2,43 g Polysaccharid-Konzentrat werden erhalten.

Beispiel 5

200 g getrocknete Lindenblüten werden mit 1500 ml Wasser nass desintegriert. Dann wird das Gemisch bei 20 °C 3 Stunden lang stehen gelassen. Nach dem Filtrieren wird der wässrige Auszug auf die im Beispiel 1 beschriebene Weise aufgearbeitet. 1,86 g Polysaccharid-Konzentrat werden erhalten.

Beispiel 6

200 g frische Lindenblüten werden auf die im Beispiel 3 beschriebene Weise aufgearbeitet. 0,62 g Polysaccharid-Konzentrat werden erhalten.

Beispiel 7

2000 g getrocknete Lindenblüten werden gemahlen. Das Mahlgut wird in 5000 ml 60%igem Methanol bei 20 °C 24 Stunden lang stehen gelassen. Dann wird der Feststoff durch Pressen und Filtrieren abgetrennt und bei Raumtemperatur getrocknet. Zu dem getrockneten Material werden 28 Liter Wasser gegeben, das Gemisch bei 20 °C 24 Stunden lang gerührt und dann die wässrige Phase durch Zentrifugieren entfernt. Zu der wässrigen Phase werden unter Rühren 56 Liter Äthanol gegeben. Der ausgefallene Niederschlag wird abfiltriert, mit Äthanol gewaschen und dann getrocknet. 47,18 g Polysaccharid-Konzentrat werden erhalten. Das Konzentrat wird in 500 ml Wasser aufgelöst und durch erneutes Ausfällen mit 1000 ml Methanol gereinigt. 32,64 g gereinigtes Polysaccharid-Konzentrat werden erhalten.

### Beispiel 8

7,8 g frische Quittenkerne werden bei +4°C 24 Stunden lang in 50 ml Wasser eingeweicht. Dann wird das Gemisch filtriert, und zu der wässrigen Phase werden unter Rühren 100 ml Methanol gegeben. Der ausgefallene Niederschlag wird abfiltriert, mit Methanol gewaschen und dann getrocknet. 2,4 g Polysaccharid-Konzentrat werden erhalten.

### Beispiel 9

200 g trockene Leinsaat werden mit 500 ml Wasser bei +36°C 2 Stunden lang extrahiert. Nach dem Filtrieren werden zu dem wässrigen Teil 1000 ml Methanol gegeben. Der ausgefallene Niederschlag wird abgetrennt, mit Methanol gewaschen und dann getrocknet. 6,79 g Polysaccharid-Konzentrat werden erhalten.

### Beispiel 10

200 g Wurzeln der weissen Malve werden in frischem Zustand in Stücke zerteilt und bei 20°C 4 Stunden lang in 400 ml Wasser eingeweicht. Der wässrige Teil wird durch Zentrifugieren abgetrennt und unter Rühren mit 800 ml Äthanol versetzt. Der ausgefallene Niederschlag wird abfiltriert, mit Äthanol gewaschen und dann getrocknet. 0,34 g Polysaccharid-Konzentrat werden erhalten.

### Beispiel 11

32 kg frische Kitaibela vitifolia herba werden in 30 Liter Wasser zerkleinert, dann wird der Brei unter einem Druck von 23 MPaü [230 atü] ausgepresst. 50 Liter Presssaft werden erhalten. Der Presssaft wird auf 75°C erwärmt, dann wird das ausgefallene Eiweiss durch Zentrifugieren entfernt. Die flüssige Phase wird unter vermindertem Druck auf 2 Liter Volumen eingeengt. Zu diesem Restvolumen werden unter Rühren 14 Liter Methanol gegeben. Das Gemisch wird 24 Stunden lang stehen gelassen. Der Niederschlag wird durch Zentrifugieren abgetrennt, mit Methanol gewaschen und bei 60°C getrocknet. 87,0 g Polysaccharid-Konzentrat werden erhalten.

### Beispiel 12

1 kg Samen von Trigonella foenum-graecum (Bockshornklee) wird in einer Hammermühle durch einen 2 mm Rost hindurch zerkleinert. Das Mahlgut wird in 8,7 l Leitungswasser bei 23°C 6 Stunden lang gerührt. Der wässrige Auszug wird anschliessend in einer Absetzzentrifuge abgetrennt. 5 Liter wässrige Lösung werden erhalten, diese wird auf 72°C erwärmt, wobei die Eiweisse ausfallen; diese werden nach dem Abkühlen durch Filtrieren entfernt. Dem Filtrat werden unter Rühren 5 Liter n-Propanol zugesetzt. Das Gemisch wird bei Raumtemperatur 13 Stunden lang stehen gelassen und dann zentrifugiert. Der Niederschlag wird mit 0,5 Liter n-Propanol vermischt in einem wässrigen Medium desintegriert und dann erneut zentrifugiert. Die abgetrennte Festsubstanz wird unter vermindertem Druck bei 45°C getrocknet. 50,0 g Polysaccharid-Konzentrat werden erhalten.

### Beispiel 13

1 kg von der Schale und den Kernen gesäuberter Kürbis (Cucurbitae maximae fructus) wird bei 20°C mit 1,5 Liter Leitungswasser im Desintegrator behandelt. Der erhaltene Brei wird in einer hydraulischen Presse unter einem Druck von 13 MPaü [130 atü] ausgepresst. Der Presssaft wird durch eine Filterplatte Seitz K-5 filtriert. 1500 ml Filtrat werden erhalten. Dieses wird bei 40°C unter vermindertem Druck auf 40% Trockensubstanzgehalt eingedickt. Zu dem Restvolumen von 140 ml werden unter Rühren 300 ml 96%iger Äthylalkohol gegeben. Das niederschlagshaltige Gemisch wird bei Raumtemperatur 12 Stunden lang stehen gelassen. Die Festsubstanz wird abfiltriert, mit 100 ml 96%igem Äthylalkohol verrieben, filtriert und unter vermindertem Druck eingedampft. 3,0 g Polysaccharid-Konzentrat werden erhalten.

### Beispiel 14

200 g getrocknete Malvenblätter werden mit 1 Liter Wasser extrahiert. Der wässrige Extrakt mit den Malvenblättern wird 2 Stunden lang stehen gelassen und dann durch Auspressen der wässrigen Phase, Zentrifugieren des Presssaftes und Filtrieren aufgearbeitet. Zu dem klaren wässrigen Extrakt werden unter Rühren 2 Liter Methylalkohol gegeben. Der ausgefallene Niederschlag wird abfiltriert und mit Methanol gewaschen. 2,0 g Polysaccharid-Konzentrat werden erhalten.

Werden als Ausgangsstoff nicht getrocknete Malvenblätter, sondern getrocknete und gemahlene Malvenblüten in der gleichen Menge eingesetzt, so erhält man 3,5 g Polysaccharid-Konzentrat.

Geht man von 200 g der getrockneten und gemahlenen Färbermalve aus, so erhält man 1,0 g Polysaccharid-Konzentrat.

### Beispiel 15

200 g getrocknete und gemahlene Wegerichblätter werden auf die im Beispiel 14 beschriebene Weise aufgearbeitet. Man erhält 0,5 g Polysaccharid-Konzentrat.

### Beispiel 16

200 g Samen des Flohkrautes werden mit 500 ml Wasser extrahiert. Im weiteren geht man gemäss Beispiel 14 vor. 10,0 g Polysaccharid-Konzentrat werden erhalten.

### Beispiel 17

200 g getrocknete Lindenblüten werden auf die im Beispiel 5 beschriebene Weise extrahiert. Zu dem klaren wässrigen Extrakt werden unter Rühren 30 g Bariumhydroxyd gegeben. Der ausgefallene Niederschlag wird abfiltriert und zuerst mit Alkohol, dann mit wässriger Natriumsulfatlösung gewaschen. 2,5 g Polysaccharid-Konzentrat werden erhalten.

Das Beispiel wird wiederholt, jedoch wird statt Bariumhydroxyd Ammoniumsulfat in einer Menge von 45 g verwendet. Der Niederschlag wird abfiltriert und mit Alkohol gewaschen. 2,0 g Polysaccharid-Konzentrat werden erhalten.

Beispiel 18

Das Gemisch von 200 g getrockneten und gemahlenen Malvenblüten und 200 g getrockneten und gemahlenen Wegerichblättern wird mit 2 Liter Wasser extrahiert. Der Extrakt wird gemäss Beispiel 14 aufgearbeitet. 2,7 g Polysaccharid-Konzentrat werden erhalten.

Beispiel 19

Ein 10 kg schwerer Kürbis (Cucurbitae maximae fructus), der, 15 Gew.-% Trockensubstanz, 4 Gew.-% reduzierende und 5 Gew.-% nicht reduzierende Zucker sowie 3,0 Gew.-% Polysaccharide enthält, wird aufgeschnitten und von den Kernen gesäubert. Der Kürbis wird im Desintegrator zu Brei verarbeitet, dieser wird mit 10 Liter Wasser verdünnt. Das Gemisch wird unter Rühren auf 35 °C erwärmt und unter lebhaftem Rühren eine Stunde lang auf dieser Temperatur gehalten. Nach 6stündigem Abstehen wird der Brei ausgepresst. 12 Liter Presssaft werden erhalten. Der Pressrückstand wird in 10 Liter Leitungswasser suspendiert, eine Stunde lang gerührt und dann erneut ausgepresst. Wieder werden 12 Liter Presssaft erhalten.

Die Presssäfte werden vereinigt und unter Rühren auf 60 °C erwärmt. Der ausgefallene Eiweissniederschlag wird durch Zentrifugieren entfernt. Die zentrifugierte Lösung wird durch ein Filterpapier Seitz K-5 filtriert, der Niederschlag wird verworfen. Die klare Lösung wird unter vermindertem Druck bei 60 °C auf 10–20% Trockensubstanzgehalt eingeengt. Das auf Raumtemperatur abgekühlte Restvolumen wird zentrifugiert, der Niederschlag wird verworfen. Zu der Lösung wird das doppelte Volumen Methanol gegeben. Die niederschlagshaltige Lösung wird bei Raumtemperatur 24 Stunden lang stehen gelassen. Die klare Oberphase wird dekantiert und abgegossen. Die den Niederschlag enthaltende Lösung wird zentrifugiert. Der Niederschlag wird in 500 ml Methanol desintegriert und dann zentrifugiert. Nach dem Zentrifugieren wird der Niederschlag noch zweimal mit Methanol gewaschen. Nach dem dritten Desintegrieren des Niederschlages wird die Lösung mit dem Niederschlag auf eine Fritte gegeben und die Mutterlauge abgesaugt.

Der nutschenfeuchte weisse Niederschlag wird mit der 10fachen Menge destillierten Wassers desintegriert. Die erhaltene Lösung wird unter Rühren auf 75 °C erwärmt und noch warm zentrifugiert. Zu der gereinigten Lösung wird unter Rühren das dreifache Volumen Methylalkohol gegeben. Das Gemisch wird bei Raumtemperatur 24 Stunden lang stehen gelassen. Danach wird zentrifugiert, der Niederschlag in 300 ml Methanol desintegriert und erneut zentrifugiert. Diese Wäsche mit Methanol wird noch zweimal wiederholt. Nach der dritten Wäsche wird der Niederschlag auf ein Vakuumsaugfilter aufgebracht, abgesaugt und bei 40 °C im Vakuum getrocknet. 21,1 g weisses, pulverförmiges Polysaccharid-Konzentrat werden erhalten.

Zwecks weiterer Reinigung wird das Polysaccharid bei 50 °C in 600 ml destilliertem Wasser gelöst, die Lösung wird eine Stunde lang stehen gelassen und dann zentrifugiert. Die klare Lösung wird unter 0,5 MPaü [5 atü] Druck durch ein Membranfilter filtriert (Diaflo XM 300®, Hersteller: Amicon Co., USA), welches die Verbindungen mit einem Molekulargewicht von über 75 000 zurückhält. Auf der hochmolekularen Seite des Membranfilters wird die Lösung mit dem dreifachen Volumen destilliertem Wasser verdünnt, dann wird der Druckfiltriervorgang wiederholt. Schliesslich wird die auf der hochmolekularen Seite des Filters gebliebene Lösung zentrifugiert und die erhaltene klare Lösung unter Rühren mit der dreifachen Menge Methanol versetzt. Das Gemisch wird bei Raumtemperatur 24 Stunden lang stehen gelassen. Der Niederschlag wird auf dem Vakuumfilter abgesaugt und bei 40 °C im Vakuum getrocknet. 13,2 g gereinigtes Polysaccharid-Konzentrat werden erhalten.

Beispiel 20

10 kg Samen des Bockshornklees (Trigonella foenum-graecum) werden in einer mit einer 3 mm Rosteinlage versehenen Hammermühle zerkleinert. Das Mahlgut wird in 40 Liter Wasser der Temperatur 35 °C eingerührt, das Gemisch 6 Stunden lang gerührt und dann in einem Scheider die flüssige von der festen Phase getrennt. Die Extraktion wird mit 30 Litern Leitungswasser der Temperatur von 35 °C 3 Stunden lang wiederholt.

Die vereinigten wässrigen Extrakte werden auf 60 °C erwärmt und dann durch Zentrifugieren und Filtrieren gereinigt. Zu der flüssigen Phase wird das doppelte Volumen 96%igen Äthylalkohols gegeben und dabei das Gemisch intensiv gerührt. Die Aufarbeitung erfolgt auf die im Beispiel 19 angegebene Weise. 180 g Polysaccharid-Konzentrat werden erhalten.

Nach saurer Hydrolyse können Galaktose- und Mannosezucker nachgewiesen werden.

Der aliquote Teil der gemäss den Beispielen 1 bis 20 hergestellten Polysaccharidkonzentrate wurde auf eine mit Sephadex G 75® gefüllte Säule aufgegeben und einer Analyse unterzogen. Die folgenden Ergebnisse wurden erhalten, wobei jeweils die weiter oben angegebenen Verfahrensweisen angewandt wurden:

Molekulargewicht:

75 000 bis 2 000 000.

Stickstoffgehalt:

höchstens 5 Gew.-%.

Phosphorgehalt, ausgedrückt als Phosphorpentoxyd:

höchstens 5 Gew.-%.

Glührückstand:

höchstens 25 Gew.-%.

Zuckergehalt:

mindestens 60 Gew.-%, davon mindestens 30 Gew.-% reduzierende Zucker.

Nach saurer Hydrolyse konnten mindestens 2 von den Bestandteilen Glucose, Galaktose, Xylose, Rhamnose, Arabinose und Uronsäure(n) nachgewiesen werden. Die im Carraghenin-Ödem-Versuch an Rattenpfoten gemessene entzündungshemmende Wirkung war mit der Wirkung von 4-Butyl-1,2-di-(phenyl)-pyrazolidin-3,5-dion {Phenylbutazon} und 1-[4'-(Chlor)-benzoyl]-5-[methoxy]-2-[3''-(methyl)-indolyl]-essigsäure {Indometacin} grössenordnungsmässig vergleichbar. Die an Mäusen bestimmten peroralen $LD_{50}$-Werte lagen über 3 000 mg/kg Körpergewicht.

Beispiel 21

Zur Herstellung von entzündungshemmenden Zäpfchen (Suppositorien) wird das gemäss Beispiel 19 hergestellte Polysaccharidkonzentrat auf Teilchengrössen von 1 bis 5 µ zerkleinert. Die Zäpfchen werden aus 5 bis 30 Gew.-% Kakaobutter (Butirum cacao) enthaltendem Lanolin (Adeps solidus) [Pharmacopea Hungarica VI., Ed. VII. Tom. 1.] bei 40 °C gegossen. Für jedes der 3 g schweren Zäpfchen wird 0,5 g des mikronisierten Polysaccharides verwendet.

Beispiel 22

Zur Herstellung eines äusserlich anzuwendenden Geles wird eine 3 gew.-%-ige wässrige Lösung von Carbopol 934® mit einer n Natronlauge auf einen pH-Wert von 7,0 eingestellt. Zur wässrigen Lösung sind vorher 3 Gew.-% des gemäss dem Beispiel 19 hergestellten Polysaccharidkonzentrates zugegeben worden. Das Gel wird mit 0,5 Gew.-% Sorbinsäure oder 0,1 Gew.-% Oxybenzoesäuremethylester [Nipagin M®] konserviert.

Beispiel 23

Zur Herstellung einer Salbe werden die im Beispiel 22 verwendeten Konserviermittel und 5 Gew.-% des gemäss dem Beispiel 20 hergestellten Polysaccharidkonzentrates in Wasser gelöst. Die Lösung wird zu 1 000 g Unguentum emulsificans (Zusammensetzung: 100 g Carboxäthanstearat, 100 g Paraffin, 300 g Cetylalkoholstearat und 500 g Vaselinum album) zugegeben und damit homogenisiert.

Beispiel 24

Zur Herstellung einer peroral verabreichbaren Suspension werden 5 g des gemäss dem Beispiel 20 hergestellten Polysaccharidkonzentrates in 100 g Wasser gelöst. Zur Lösung werden 0,12 g Sorbinsäure, 2,8 g Magnesiumoxyd, 8 g Aluminiumhydroxyd, 60 g Methylcellulosehydrogel und 20 g Sorbit zugegeben. Das Gemisch wird mit doppelt destilliertem Wasser auf 200 g aufgefüllt.

Beispiel 25

Zur Herstellung von peroral verabreichbaren Tabletten werden 0,25 g des gemäss dem Beispiel 19 hergestellten Polysaccharidkonzentrates mit 0,25 g Granulatum simplex der folgenden Zusammensetzung zu Tabletten gepresst:

60 Gew.-Teile Milchzucker
16 Gew.-Teile Rohrzucker
20 Gew.-Teile Kartoffelstärke
 2 Gew.-Teile Talk
 2 Gew.-Teile Magnesiumstearat.

**Patentansprüche für die Vertragsstaaten BE, CH, DE, FR, GB, IT, LI, LU, NL, SE**

1. Polysaccharidkonzentrate pflanzlichen Ursprungs mit

a) einem Molekulargewicht von 75 000 bis 2 000 000,

b) einem Gehalt an

α) höchstens 5 Gew.-% Stickstoff (Mikrokjeldahl-Verfahrensweise nach Wagner-Parnass; ungarische Norm 6830–66) und

β) höchstens 5 Gew.-% Phosphor, ausgedrückt als Phosphorpentoxyd, (mit Molybdänat-Eikonogen-Reagenz; Peac-Tracey: Moderne Methoden der Pflanzenanalyse, Band IV, Seite 308, Springer-Verlag, 1955, Berlin), die

c) höchstens 25 Gew.-% Glührückstand hinterlassen,

d)

α) mindestens 30 Gew.-% reduzierende Zucker (nach saurer Hydrolyse mit o-Toluidin bestimmt; R. Richterich: Klinische Chemie 1968, Seite 233, Carger Ed. Basel-New York) enthalten und

β) deren Gesamtzuckergehalt mindestens 60 Gew.-% (nach der Phenol-Schwefelsäure-Verfahrensweise bestimmt; M. Dubois: Anal. Chem. 28 [1956], Seite 35) beträgt, wobei

e) sie praktisch keine Stoffe mit Molekulargewichten unter 75 000 enthalten (untersucht mit einem Molekularsieb, bestehend aus einem dreidimensional vernetzten Polysaccharid, das durch Quervernetzung der linearen Makromoleküle von Dextran erhalten worden ist, mit etwa mittlerem Vernetzungsgrad,) und

f) die nach saurer Hydrolyse mindestens 2 von den Bestandteilen Glucose, Galaktose, Xylose, Rhamnose, Arabinose und Uronsäure(n) enthalten,

erhalten durch Behandeln von Polysaccharid enthaltenden Drogenpflanzen mit die Polysaccharide lösenden Mitteln, wobei man 1 oder mehr frische oder getrocknete, Polysaccharide enthaltende Drogenpflanzen, 1 bis 24 Stunden lang bei 0 bis 40 °C mit Wasser behandelt beziehungsweise extrahiert und danach oder dabei [jeweils] die Feststoffe entfernt und dann aus dem beziehungsweise den wässrigen Auszug beziehungsweise Auszügen {flüssigen Teil(en)} durch Zugabe von 1 oder mehr Alkohol(en) mit 1 bis 3 Kohlenstoffatom(en), Aceton und/oder 1- bis 3-wertigen Kationen des Polysaccharidkonzentrat fällt und dieses von der wässrigen Phase abtrennt, dadurch gekennzeichnet, dass sie gewonnen werden durch Aufarbeitung von 1 oder mehr Pflanzen der folgenden Pflanzenfamilien: Kürbis (Cucurbitaceae), Schmetterlingsblütler (Papilionaceae), lindenartige Gewächse (Tiliaceae), Lippenblütler (Labiatae), Wurzeln der weissen Malve (Althaeae radix), Malvenblatt oder -blüte (Malvae folium et flos), Kraut der Färbermalve (Malvae arboreae herba), Korbblütler (Compositae beziehungsweise Asteraceae), Doldenblütler (Umbellifereae), Rautengewächse (Rutaceae), Gänsefussgewächse (Chenopodiaceae), leinartige Gewächse (Linaceae), Rosengewächse (Rosaceae) und wegerichartige Gewächse (Plantaginaceae), wobei diese Polysaccharidkonzentrate

g) im Carraghenin-Ödem-Versuch an Rattenpfoten eine mit der Wirkung von 4-Butyl-1,2-di-(phenyl)-pyrazolidin-3,5-dion {Phenylbutazon} und 1-[4'-(Chlor)-benzoyl]-5-[methoxy]-2-[3''-(methyl)-indolyl]-essigsäure {Indometacin} vergleichbare entzündungshemmende Wirkung zeigen und

h) ihr peroral bestimmter $LD_{50}$-Wert an Mäusen grösser als 3 000 mg/kg Körpergewicht und an Ratten grösser als 2 000 mg/kg Körpergewicht ist.

2. Polysaccharidkonzentrate nach Anspruch 1, dadurch gekennzeichnet, dass sie gewonnen werden durch Aufarbeiten von 1 oder mehr der folgenden Pflanzen bzw. Pflanzenteile: Kamillenblüte (Chamomillae flos), Lindenblüte (Tiliae flos), Wegerichblatt (Plantaginis folium), Flohkrautsamen (Psyllii semen), Quittensamen beziehungsweise -kern (Cydoniae semen), Leinsaat (Lini semen), Bockshornkleesamen (Trigonella foeni-graeci semen), Kürbis (Cucurbitae maximae fructus) und Kitaibela vitifolia herba.

3. Polysaccharidkonzentrate nach Anspruch 1 oder 2, dadurch gekennzeichnet, dass sie gewonnen werden aus Kürbis (Cucurbita maxima), ihr Molekulargewicht grösser als 300 000 ist, sie höchstens 3 Gew.-% Stickstoff enthalten, höchstens 20 Gew.-% Glührückstand hinterlassen und nach saurer Hydrolyse vor allem Glucose, Galaktose und Uronsäuren enthalten und in einer Dosis von 10 mg/kg Körpergewicht ihre entzündungshemmende Wirkung im Carraghenin-Ödem-Versuch an Rattenpfoten mindestens 50% beträgt.

4. Polysaccharidkonzentrate nach Anspruch 1 oder 2, dadurch gekennzeichnet, dass sie gewonnen werden aus Bockshornkleesamen (Trigonella foeni-graeci semen), ihr Molekulargewicht grösser als 100 000 ist, sie höchstens 3 Gew.-% Stickstoff und höchstens 4 Gew.-% Phosphor, ausgedrückt als Phosphorpentoxyd, enthalten, höchstens 5 Gew.-% Glührückstand hinterlassen, mindestens 50 Gew.-% reduzierende Zucker und mindestens 70 Gew.-% Gesamtzucker enthalten und nach saurer Hydrolyse vor allem Galaktose und Mannose enthalten und in einer Dosis von 10 mg/kg Körpergewicht ihre entzündungshemmende Wirkung im Carraghenin-Ödem-Versuch an Rattenpfoten mindestens 40% beträgt.

5. Polysaccharidkonzentrate nach den Ansprüchen 1 bis 4, dadurch gekennzeichnet, dass sie in der Weise gewonnen werden, dass man zum Fällen des Polysaccharidkonzentrates den beziehungsweise die Alkohol(e) im 1- bis 7-fachen Volumen und das Aceton im bis zu 10-fachen Volumen, jeweils bezogen auf den wässrigen Auszug, und die 1- bis 3-wertigen Kationen in der $1/20$-sten bis $1/5$-ten Menge, bezogen auf das Gewicht der Drogenpflanzen, verwendet.

6. Polysaccharidkonzentrate nach den Ansprüchen 1 bis 5, dadurch gekennzeichnet, dass sie unter Verwendung von solchen Polysaccharide enthaltenden Drogenpflanzen gewonnen werden, welche mit 1 oder mehr organischen Lösungsmittel(n) vorbehandelt worden sind.

7. Polysaccharidkonzentrate nach den Ansprüchen 1 bis 6, dadurch gekennzeichnet, dass sie gewonnen werden unter Verwendung von solchen Polysaccharide enthaltenden Drogenpflanzen, zu deren Vorbehandlung als organische(s) Lösungsmittel (ein) wässrige(s) eingesetzt worden ist bzw. sind.

8. Polysaccharidkonzentrate nach den Ansprüchen 1 bis 7, dadurch gekennzeichnet, dass sie gewonnen werden unter Verwendung von solchen Polysaccharide enthaltenden Drogenpflanzen, deren Vorbehandlung mit dem bzw. den organischen Lösungsmittel(n) nach einem Desintegrieren durchgeführt worden ist.

9. Polysaccharidkonzentrate nach den Ansprüchen 1 bis 8, dadurch gekennzeichnet, dass sie in der Weise gewonnen werden, dass man die Behandlung bzw. das Extrahieren mit dem Wasser mehrmals durchführt.

10. Polysaccharidkonzentrate nach den Ansprüchen 1 bis 9, dadurch gekennzeichnet, dass sie in der Weise gewonnen werden, dass man die Behandlung(en) bzw. das bzw. die Extraktion(en) mit dem Wasser nach einem oder während eines Desintergrieren(s) durchführt.

11. Polysaccharidkonzentrate nach den Ansprüchen 1 bis 10, dadurch gekennzeichnet, dass sie in der Weise gewonnen werden, dass man zur bzw. zu den Behandlung(en) mit Wasser die 1- bis 20-fache Wassermenge, bezogen auf das Gewicht der Drogenpflanze, verwendet.

12. Polysaccharidkonzentrate nach den Ansprüchen 1 bis 11, dadurch gekennzeichnet, dass sie in der Weise gewonnen werden, dass man das Behandeln bzw. Extrahieren mit Wasser mehrmals durchführt.

13. Polysaccharidkonzentrate nach den Ansprüchen 1 bis 12, dadurch gekennzeichnet, dass sie in der Weise gewonnen werden, dass man die Behandlung(en) mit dem Wasser und das Abtrennen der Feststoffe in der Weise durchführt, dass man nach dem Desintegrieren der frischen Drogenpflanze(n) oder der getrockneten Drogenpflanze(n) und Auspressen des erhaltenen Breies den Pressrückstand [erneut] mit Wasser behandelt und erneut auspresst.

14. Polysaccharidkonzentrate nach den Ansprüchen 1 bis 13, dadurch gekennzeichnet, dass sie in der Weise gewonnen werden, dass man als frische oder getrocknete Drogenpflanze(n) [eine] gewässerte verwendet.

15. Polysaccharidkonzentrate nach den Ansprüchen 1 bis 14, dadurch gekennzeichnet, dass sie in der Weise gewonnen werden, dass man das Fällen des Polysaccharidkonzentrates mit dem bzw. den Alkohol(en), Aceton und/oder 1- bis 3-wertigen Kationen nach Abtrennen der einweissartigen Stoffe und/oder Einengen auf ein geringes Volumen durchführt.

16. Polysaccharidkonzentrate nach den Ansprüchen 1 bis 15, dadurch gekennzeichnet, dass sie in der Weise gewonnen werden, dass man das Entfernen der im wässrigen Auszug befindlichen eiweissartigen Stoffe in der Weise durchführt, dass man den wässrigen Auszug auf 40 bis 80 °C erwärmt und den ausgefallenen Niederschlag abtrennt.

17. Polysaccharidkonzentrate nach den Ansprüchen 1 bis 16, dadurch gekennzeichnet, dass sie in der Weise gewonnen werden, dass man das Abtrennen des Eiweissniederschlags durch Zentrifugieren und/oder Filtrieren durchführt.

18. Polysaccharidkonzentrate nach den Ansprüchen 1 bis 17, dadurch gekennzeichnet, dass sie in der Weise gewonnen werden, dass man zum Fällen des Polysaccharidkonzentrates den bzw. die Alkohol(e) im 1- bis 3-fachen Volumen, bezogen auf den wässrigen Auszug, verwendet.

19. Polysaccharidkonzentrate nach den Ansprüchen 1 bis 18, dadurch gekennzeichnet, dass sie in der Weise gewonnen werden, dass man das Abtrennen des Polysaccharidkonzentrats von der wässrigen Phase nach einem Stehenlassen durch Zentrifugieren und/oder Filtrieren durchführt, worauf man es mit dem Fällungsmittel wäscht.

20. Polysaccharidkonzentrate nach den Ansprüchen 1 bis 19, dadurch gekennzeichnet, dass sie in der Weise gewonnen werden, dass man das Waschen des abgetrennten Polysaccharidkonzentrats mehrmals durchführt.

21. Polysaccharidkonzentrate nach den Ansprüchen 1 bis 20, dadurch gekennzeichnet, dass sie in der Weise gewonnen werden, dass man das erhaltene Polysaccharidkonzentrat reinigt.

22. Polysaccharidkonzentrate nach den Ansprüchen 1 bis 21, dadurch gekennzeichnet, dass sie in der Weise gewonnen werden, dass man das Reinigen des Polysaccharidkonzentrats durch Umfällen durchführt.

23. Polysaccharidkonzentrate nach den Ansprüchen 1 bis 22, dadurch gekennzeichnet, dass sie in der Weise gewonnen werden, dass man das Umfällen mehrmals durchführt.

24. Polysaccharidkonzentrate nach den Ansprüchen 1 bis 23, dadurch gekennzeichnet, dass sie in der Weise gewonnen werden, dass man das Reinigen durch Umfällen des 1-mal gefällten rohen Polysaccharidkonzentrates in der Weise durchführt, dass man es in Wasser löst und mit einem zum ersten Fällen verwendbaren Fällungsmittel fällt, wobei man die Reihenfolge der Zugabe des Wassers und Fällungsmittels beliebig wählen kann.

25. Polysaccharidkonzentrate nach den Ansprüchen 1 bis 24, dadurch gekennzeichnet, dass sie in der Weise gewonnen werden, dass man das Reinigen durch Umfällen des 1-mal gefällten rohen Polysaccharidkonzentrates in der Weise durchführt, dass man es in der 5- bis 15-fachen Menge Wasser, bezogen auf sein Gewicht, löst und auf 65 bis 80 °C erwärmt, die ausgefallenen Substanzen durch Zentrifugieren entfernt, aus der klaren Lösung das Polysaccharidkonzentrat durch Zugabe eines zum ersten Fällen verwendbaren Fällungsmittels fällt, das Gemisch stehen lässt und dann den Niederschlag durch Zentrifugieren und/oder Filtrieren abtrennt, mit dem Fällungsmittel wäscht und unter Vakuum bei höchstens 60 °C trocknet.

26. Polysaccharidkonzentrate nach den Ansprüchen 1 bis 25, dadurch gekennzeichnet, dass sie in der Weise gewonnen werden, dass man das Umfällen mit einem Alkohol durchführt.

27. Polysaccharidkonzentrate nach den Ansprüchen 1 bis 26, dadurch gekennzeichnet, dass sie in der Weise gewonnen werden, dass man das Umfällen 1-mal oder mehrmals wiederholt.

28. Polysaccharidkonzentrate nach den Ansprüchen 1 bis 27, dadurch gekennzeichnet, dass sie in der Weise gewonnen werden, dass man das Trocknen bei höchstens 40 °C durchführt.

29. Polysaccharidkonzentrate nach den Ansprüchen 1 bis 28, dadurch gekennzeichnet, dass sie in der Weise gewonnen werden, dass man das Reinigen bzw. Weiterreinigen des erhaltenen rohen bzw. gereinigten Polysaccharidkonzentrates in der Weise durchführt, dass man es in der 25- bis 40-fachen Menge Wasser, bezogen auf sein Gewicht, bei 50 bis 75 °C löst, die Lösung durch Zentrifugieren reinigt und dann unter Überdruck durch ein die Stoffe mit einem Molekulargewicht unter 75 000 durchlassendes Membranfilter drückt und aus der auf der hochmolekularen Seite des Membranfilters gebliebenen Lösung mit einem zum ersten Fällen verwendbaren Fällungsmittel das Polysaccharid fällt.

30. Polysaccharidkonzentrate nach den Ansprüchen 1 bis 29, dadurch gekennzeichnet, dass sie in der Weise gewonnen werden, dass man die auf der hochmolekularen Seite des Membranfilters gebliebene Lösung vor dem Fällen des Polysaccharides aus ihr mit Wasser verdünnt.

31. Polysaccharidkonzentrate nach den Ansprüchen 1 bis 30, dadurch gekennzeichnet, dass sie in der Weise gewonnen werden, dass man das Fällen des Polysaccharides aus der auf der hochmolekularen Seite des Membranfilters gebliebenen Lösung mit einem Alkohol durchführt.

32. Polysaccharidkonzentrate nach den Ansprüchen 1 bis 31, dadurch gekennzeichnet, dass sie in der Weise gewonnen werden, dass man das Lösen in Wasser und Reinigen durch Zentrifugieren und/oder Membranfiltrieren und/oder Fällen 1-mal oder mehrmals wiederholt.

33. Arzneimittel, gekennzeichnet durch einen Gehalt an 1 oder mehr Polysaccharidkonzentrat(en) nach den Ansprüchen 1 bis 32 als Wirkstoffen.

34. Arzneimittel nach Anspruch 33, dadurch gekennzeichnet, dass sie das bzw. die Polysaccharidkonzentrat(e) zusammen mit 1 oder mehr anderen nicht-steroiden entzündungshemmenden Stoff(en) und/oder 1 oder mehr üblichen pharmazeutischen Träger- und/oder Hilfsstoff(en) enthalten.

35. Kosmetische Präparate, gekennzeichnet durch einen Gehalt an 1 oder mehr Polysaccharidkonzentrat(en) nach den Ansprüchen 1 bis 32 als Wirkstoffen.

36. Kosmetische Präparate nach Anspruch 35, dadurch gekennzeichnet, dass sie das bzw. die Polysaccharidkonzentrat(e) zusammen mit 1 oder mehr üblichen äusserlich anwendbaren Träger- und/oder Hilfsstoff(en) enthalten.

**Patentansprüche für den Vertragsstaat: AT**

1. Verfahren zur Herstellung von Polysaccharidkonzentraten pflanzlichen Ursprungs mit
   a) einem Molekulargewicht von 75 000 bis 2 000 000,
   b) einem Gehalt an
   α) höchstens 5 Gew.-% Stickstoff (Mikrokjeldahl-Verfahrensweise nach Wagner-Parnass; ungarische Norm 6830–66) und
   β) höchstens 5 Gew.-% Phosphor, ausgedrückt als Phosphorpentoxyd, (mit Molybdänat-Eikonogen-Reagenz; Peac-Tracey: Moderne Methoden der Pflanzenanalyse, Band IV, Seite 307, Springer-Verlag, 1955, Berlin), die
   c) höchstens 25 Gew.-% Glührückstand hinterlassen,
   d)
   α) mindestens 30 Gew.-% reduzierende Zucker (nach saurer Hydrolyse mit o-Toluidin bestimmt; R. Richterich: Klinische Chemie 1968, Seite 233, Carger Ed. Basel-New York) enthalten und
   β) deren Gesamtzuckergehalt mindestens 60 Gew.-% (nach der Phenol-Schwefelsäure-Verfahrensweise bestimmt; M. Dubois: Anal. Chem. 28 1965, Seite 35) beträgt, wobei
   e) sie praktisch keine Stoffe mit Molekulargewichten unter 75 000 enthalten (untersucht mit einem Molekularsieb, bestehend aus einem dreidimensional vernetzten Polysaccharid, das durch Quervernetzung der linearen Makromoleküle von Dextran erhalten worden ist, mit etwa mittlerem Vernetzungsgrad,) und
   f) die nach saurer Hydrolyse mindestens 2 von den Bestandteilen Glucose, Galaktose, Xylose, Rhamnose, Arabinose und Uronsäure(n) enthalten,
   g) wobei sie im Carraghenin-Ödem-Versuch an Rattenpfoten eine mit der Wirkung von 4-Butyl-1,2-di-(phenyl)-pyrazolidin-3,5-dion {Phenylbutazon} und 1-[4′-(Chlor)-benzoyl]-5-[methoxy]-2-[3″-(methyl)-indolyl]-essigsäure {Indometacin} vergleichbare entzündungshemmende Wirkung zeigen und
   h) ihr peroral bestimmter $LD_{50}$-Wert an Mäusen grösser als 3 000 mg/kg Körpergewicht und an Ratten grösser als 2 000 mg/kg Körpergewicht ist, wobei diese Polysaccharidkonzentrate erhalten werden durch Behandeln von Polysaccharid enthaltenden Drogenpflanzen mit die Polysaccharide lösenden Mitteln, wobei man 1 oder mehr frische oder getrocknete, Polysaccharide enthaltende Drogenpflanzen, 1 bis 24 Stunden lang bei 0 bis 40 °C mit Wasser behandelt beziehungsweise extrahiert und danach oder dabei [jeweils] die Feststoffe entfernt und dann aus dem beziehungsweise den wässrigen Auszug beziehungsweise Auszügen {flüssigen Teil(en)} durch Zugabe von 1 oder mehr Alkohol(en) mit 1 bis 3 Kohlenstoffatom(en), Aceton und/oder 1- bis 3-wertigen Kationen des Polysaccharidkonzentrat fällt und dieses von der wässrigen Phase abtrennt, dadurch gekennzeichnet, dass sie durch Aufarbeitung von 1 oder mehr Pflanzen der folgenden Pflanzenfamilien: Kürbis (Cucurbitaceae), Schmetterlingsblütler (Papilionaceae), lindenartige Gewächse (Tiliaceae), Lippenblütler (Labiatae), Wurzeln der weissen Malve (Althaeae radix), Malvenblatt oder -blüte (Malvae folium et flos), Kraut der Färbermalve (Malvae arboreae herba), Korbblütler (Compositae beziehungsweise Asteraceae), Doldenblütler (Umbellifereae), Rautengewächse (Rutaceae), Gänsefussgewächse (Chenopodiaceae), leinartige Gewächse (Linaceae), Rosengewächse (Rosaceae) und wegerichartige Gewächse (Plantaginaceae) hergestellt werden.

2. Verfahren nach Anspruch 1, dadurch gekennzeichnet, dass die Polysaccharidkonzentrate durch Aufarbeiten von 1 oder mehr der folgenden Pflanzen bzw. Pflanzenteile: Kamillenblüte (Chamomillae flos), Lindenblüte (Tiliae flos), Wegerichblatt (Plantaginis folium), Flohkrautsamen (Psyllii semen), Quittensamen beziehungsweise -kern (Cydoniae semen), Leinsaat (Lini semen), Bockshornkleesamen (Trigonella foeni-graeci semen), Kürbis (Cucurbitae maximae fructus) und Kitaibela vitifolia herba hergestellt werden.

3. Verfahren nach Anspruch 1 oder 2, dadurch gekennzeichnet, dass die Polysaccharidkonzentrate, deren Molekulargewicht grösser als 300 000 ist, die höchstens 3 Gew.-% Stickstoff enthalten, höchstens 20 Gew.-% Glührückstand hinterlassen und nach saurer Hydrolyse vor allem Glucose, Galaktose und Uronsäuren enthalten, und wobei in einer Dosis von 10 mg/kg Körpergewicht ihre entzündungshemmende Wirkung im Carraghenin-Ödem-Versuch an Rattenpfoten mindestens 50% beträgt, aus Kürbis (Curcurbita maxima) gewonnen werden.

4. Verfahren nach Anspruch 1 oder 2, dadurch gekennzeichnet, dass die Polysaccharidkonzentrate, deren Molekulargewicht grösser als 100 000 ist, die höchstens 3 Gew.-% Stickstoff und höchstens 4 Gew.-% Phosphor, ausgedrückt als Phosphorpentoxyd, enthalten, die höchstens 5 Gew.-% Glührückstand hinterlassen, die mindestens 50 Gew.-% reduzierende Zucker und mindestens 70 Gew.-% Gesamtzucker enthalten und nach saurer Hydrolyse vor allem Galaktose und Mannose enthalten und deren entzündungshemmende Wirkung in einer Dosis von 10 mg/kg Körpergewicht im Carraghenin-Ödem-Versuch an Rattenpfoten mindestens 40% beträgt, aus Bockshornkleesamen (Trigonella foeni-graeci semen) gewonnen werden.

5. Verfahren nach den Ansprüchen 1 bis 4, dadurch gekennzeichnet, dass die Polysaccharide in der Weise gewonnen werden, dass man zum Fällen des Polysaccharidkonzentrates den beziehungsweise die Alkohol(e) im 1- bis 7-fachen Volumen und das Aceton im bis zu 10-fachen Volumen, jeweils bezogen auf den wässrigen Auszug, und die 1- bis 3-wertigen Kationen in der $1/20$-sten bis $1/5$-ten Menge, bezogen auf das Gewicht der Drogenpflanzen, verwendet.

6. Verfahren nach den Ansprüchen 1 bis 5, dadurch gekennzeichnet, die Polysaccharidkonzentrate unter Verwendung von solchen Polysaccharide enthaltenden Drogenpflanzen gewonnen werden, welche mit 1 oder mehr organischen Lösungsmittel(n) vorbehandelt worden sind.

7. Verfahren nach den Ansprüchen 1 bis 6, dadurch gekennzeichnet, die Polysaccharidkonzentrate unter Verwendung von solchen Polysaccharide enthaltenden Drogenpflanzen, zu deren Vorbehandlung als organische(s) Lösungsmittel (ein) wässrige(s) eingesetzt worden ist bzw. sind, gewonnen werden.

8. Verfahren nach den Ansprüchen 1 bis 7, dadurch gekennzeichnet, die Polysaccharidkonzentrate unter Verwendung von solchen Polysaccharide enthaltenden Drogenpflanzen, deren Vorbehandlung mit dem bzw. den organischen Lösungsmittel(n) nach einem Desintegrieren durchgeführt worden ist, gewonnen werden.

9. Verfahren nach den Ansprüchen 1 bis 8, dadurch gekennzeichnet, dass die Polysaccharidkonzentrate in der Weise gewonnen werden, dass man die Behandlung bzw. das Extrahieren mit dem Wasser mehrmals durchführt.

10. Verfahren nach den Ansprüchen 1 bis 9, dadurch gekennzeichnet, dass die Polysaccharidkonzentrate in der Weise gewonnen werden, dass man die Behandlung(en) bzw. das bzw. die Extraktion(en) mit dem Wasser nach einem oder während eines Desintegrieren(s) durchführt.

11. Verfahren nach den Ansprüchen 1 bis 10, dadurch gekennzeichnet, dass die Polysaccharidkonzentrate in der Weise gewonnen werden, dass man zur bzw. zu den Behandlung(en) mit Wasser die 1- bis 20-fache Wassermenge, bezogen auf das Gewicht der Drogenpflanze, verwendet.

12. Verfahren nach den Ansprüchen 1 bis 11, dadurch gekennzeichnet, dass die Polysaccharidkonzentrate in der Weise gewonnen werden, dass man das Behandeln bzw. Extrahieren mit Wasser mehrmals durchführt.

13. Verfahren nach den Ansprüchen 1 bis 12, dadurch gekennzeichnet, dass die Polysaccharidkonzentrate in der Weise gewonnen werden, dass man die Behandlung(en) mit dem Wasser und das Abtrennen der Feststoffe in der Weise durchführt, dass man nach dem Desintegrieren der frischen Drogenpflanze(n) oder der getrockneten Drogenpflanze(n) und Auspressen des erhaltenen Breies den Pressrückstand [erneut] mit Wasser behandelt und erneut auspresst.

14. Verfahren nach den Ansprüchen 1 bis 13, dadurch gekennzeichnet, dass die Polysaccharidkonzentrate in der Weise gewonnen werden, dass man als frische oder getrocknete Drogenpflanze(n) [eine] gewässerte verwendet.

15. Verfahren nach den Ansprüchen 1 bis 14, dadurch gekennzeichnet, dass die Polysaccharidkonzentrate in der Weise gewonnen werden, dass man das Fällen des Polysaccharidkonzentrates mit dem bzw. den Alkohol(en), Aceton und/oder 1- bis 3-wertigen Kationen, nach Abtrennen der eiweissartigen Stoffe und/oder Einengen auf ein geringes Volumen durchführt.

16. Verfahren nach den Ansprüchen 1 bis 15, dadurch gekennzeichnet, dass die Polysaccharidkonzentrate in der Weise gewonnen werden, dass man das Entfernen der im wässrigen Auszug befindlichen eiweissartigen Stoffe in der Weise durchführt, dass man den wässrigen Auszug auf 40 bis 80 °C erwärmt und den ausgefallenen Niederschlag abtrennt.

17. Verfahren nach den Ansprüchen 1 bis 16, dadurch gekennzeichnet, dass die Polysaccharidkonzentrate in der Weise gewonnen werden, dass man das Abtrennen des Eiweissniederschlags durch Zentrifugieren und/oder Filtrieren durchführt.

18. Verfahren nach den Ansprüchen 1 bis 17, dadurch gekennzeichnet, dass die Polysaccharidkonzentrate in der Weise gewonnen werden, dass man zum Fällen des Polysaccharidkonzentrates den bzw. die Alkohol(e) im 1- bis 3-fachen Volumen, bezogen auf den wässrigen Auszug, verwendet.

19. Verfahren nach den Ansprüchen 1 bis 18, dadurch gekennzeichnet, dass die Polysaccharidkonzentrate in der Weise gewonnen werden, dass man das Abtrennen des Polysaccharidkonzentrats von der wässrigen Phase nach einem Stehenlassen durch Zentrifugieren und/oder Filtrieren durchführt, worauf man es mit dem Fällungsmittel wäscht.

20. Verfahren nach den Ansprüchen 1 bis 19, dadurch gekennzeichnet, dass die Polysaccharidkonzentrate in der Weise gewonnen werden, dass man das Waschen des abgetrennten Polysaccharidkonzentrats mehrmals durchführt.

21. Verfahren nach den Ansprüchen 1 bis 20, dadurch gekennzeichnet, dass die Polysaccharidkonzentrate in der Weise gewonnen werden, dass man das erhaltene Polysaccharidkonzentrat reinigt.

22. Verfahren nach den Ansprüchen 1 bis 21, dadurch gekennzeichnet, dass die Polysaccharidkonzentrate in der Weise gewonnen werden, dass man das Reinigen des Polysaccharidkonzentrats durch Umfällen durchführt.

23. Verfahren nach den Ansprüchen 1 bis 22, dadurch gekennzeichnet, dass die Polysaccharidkonzentrate in der Weise gewonnen werden, dass man das Umfällen mehrmals durchführt.

24. Verfahren nach den Ansprüchen 1 bis 23, dadurch gekennzeichnet, dass die Polysaccharidkonzentrate in der Weise gewonnen werden, dass man das Reinigen durch Umfällen des 1-mal gefällten rohen Polysaccharidkonzentrates in der Weise durchführt, dass man es in Wasser löst und mit einem zum ersten Fällen verwendbaren Fällungsmittel fällt, wobei man die Reihenfolge der Zugabe des Wassers und Fällungsmittels beliebig wählen kann.

25. Verfahren nach den Ansprüchen 1 bis 24, dadurch gekennzeichnet, dass die Polysaccharidkonzentrate in der Weise gewonnen werden, dass man das Reinigen durch Umfällen des 1-mal gefällten rohen Polysaccharidkonzentrates in der Weise durchführt, dass man es in der 5- bis 15-fachen Menge Wasser, bezogen auf sein Gewicht, löst und auf 65 bis 80 °C erwärmt, die ausgefallenen Substanzen durch Zentrifugieren entfernt, aus der klaren Lösung das Polysaccharidkonzentrat durch Zugabe eines zum ersten Fällen verwendbaren Fällungsmittels fällt, das Gemisch stehen lässt und dann den Niederschlag durch Zentrifugieren und/oder Filtrieren abtrennt, mit dem Fällungsmittel wäscht und unter Vakuum bei höchstens 60 °C trocknet.

26. Verfahren nach den Ansprüchen 1 bis 25, dadurch gekennzeichnet, dass die Polysaccharidkonzentrate in der Weise gewonnen werden, dass man das Umfällen mit einem Alkohol durchführt.

27. Verfahren nach den Ansprüchen 1 bis 26, dadurch gekennzeichnet, dass die Polysaccharidkonzentrate in der Weise gewonnen werden, dass man das Umfällen 1-mal oder mehrmals wiederholt.

28. Verfahren nach den Ansprüchen 1 bis 27, dadurch gekennzeichnet, dass die Polysaccharidkonzentrate in der Weise gewonnen werden, dass man das Trocknen bei höchstens 40 °C durchführt.

29. Verfahren nach den Ansprüchen 1 bis 28, dadurch gekennzeichnet, dass die Polysaccharidkonzentrate in der Weise gewonnen werden, dass man das Reinigen bzw. Weiterreinigen des erhaltenen rohen bzw. gereinigten Polysaccharidkonzentrates in der Weise durchführt, dass man es in der 25- bis 40-fachen Menge Wasser, bezogen auf sein Gewicht, bei 50 bis 75 °C löst, die Lösung durch Zentrifugieren reinigt und dann unter Überdruck durch ein die Stoffe mit einem Molekulargewicht unter 75 000 durchlassendes Membranfilter drückt und aus der auf der hochmolekularen Seite des Membranfilters gebliebenen Lösung mit einem zum ersten Fällen verwendbaren Fällungsmittel das Polysaccharid fällt.

30. Verfahren nach den Ansprüchen 1 bis 29, dadurch gekennzeichnet, dass die Polysaccharidkonzentrate in der Weise gewonnen werden, dass man die auf der hochmolekularen Seite des Membranfilters gebliebene Lösung vor dem Fällen des Polysaccharides aus ihr mit Wasser verdünnt.

31. Verfahren nach den Ansprüchen 1 bis 30, dadurch gekennzeichnet, dass die Polysaccharidkonzentrate in der Weise gewonnen werden, dass man das Fällen des Polysaccharides aus der auf der hochmolekularen Seite des Membranfilters gebliebenen Lösung mit einem Alkohol durchführt.

32. Verfahren nach den Ansprüchen 1 bis 31, dadurch gekennzeichnet, dass die Polysaccharidkonzentrate in der Weise gewonnen werden, dass man das Lösen in Wasser und Reinigen durch Zentrifugieren und/oder Membranfiltrieren und/oder Fällen 1-mal oder mehrmals wiederholt.

33. Verfahren zur Herstellung eines Arzneimittels, dadurch gekennzeichnet, dass 1 oder mehrere Polysaccharidkonzentrat(e) gemäss den Ansprüchen 1 bis 32 als Wirkstoffe mit 1 oder mehr üblichen pharmazeutischen Träger- und/oder Hilfsstoff(en) vermischt werden.

34. Verfahren nach Anspruch 33, dadurch gekennzeichnet, dass das bzw. die Polysaccharidkonzentrat(e) zusammen mit 1 oder mehr anderen nicht-steroiden entzündungshemmenden Stoff(en) und/oder 1 oder mehr üblichen pharmazeutischen Träger- und/oder Hilfsstoff(en) vermischt werden.

35. Kosmetische Präparate, gekennzeichnet durch einen Gehalt an 1 oder mehr Polysaccharidkonzentrat(en) nach den Ansprüchen 1 bis 32 als Wirkstoffen.

36. Kosmetische Präparate nach Anspruch 35, dadurch gekennzeichnet, dass sie das bzw. die Polysaccharidkonzentrat(e) zusammen mit 1 oder mehr üblichen äusserlich anwendbaren Träger- und/oder Hilfsstoff(en) enthalten.

**Claims for the Contracting States BE, CH, DE, FR, GB, IT, LI, LU, NL, SE**

1. Polysaccharide concentrates which are of vegetable origin and have
   a) a molecular weight from 75,000 to 2,000,000,
   b) a content of
   α) not in excess of 5% by weight nitrogen (mikrokjeldahl procedure in accordance with Wagner-Parnass; Hungarian standard 6830–60) and
   β) not in excess of 5% by weight phosphorus, expressed as phosphorus pentoxide (with molybdenate eiconogene reagent; Peac-Tracey: Moderne Methoden der Pflanzenanalyse, Volume IV, page 308, Springer-Verlag, 1955, Berlin),
   c) a residue on ignition not in excess of 25% by weight,
   d)
   α) at least 30% by weight reducing sugars (determined after an acid hydrolysis with o-toluidine; R. Richterich: Klinische Chemie, 1968, page 233, Carger Ed. Basel - New York) and
   β) a total sugar content of at least 60% by weight (determined by the phenol-sulphuric acid procedure; M. Dubois: Anal. Chem. 28 (1956), page 35),
   e) virtually no content of substances having molecular weights below 75,000 (examined with a molecular sieve consisting of a three-dimensionally cross-linked polysaccharide, which has been obtained by a cross-linking of the linear macromolecules of dextrane, and has an approximately medium degree of cross-linking) (Sephadex G 75®) and
   f) after acid hydrolysis contain at least two of the components glucose, galactose, xylose, rhamnose, arabinose and uronic acid(s), which polysaccharide concentrates have been obtained by a treatment of polysaccharide-containing drug plants with solvents for the polysaccharides in a process in which one or more fresh or dried drug plants, which contain polysaccharides, are treated or extracted with water at 0 to 40°C for 1 to 24 hours and thereafter or during that time the respective solids are removed, followed by a precipitation of the polysaccharide concentrate from the liquid extract or extracts (liquid parts) by an addition of one or more alcohol(s) having one to three carbon atom(s), acetone and/or mono- to trivalent cations, whereafter the polysaccharide concentrate is separated from the aqueous phase, and which polysaccharide concentrates are characterized in that they are obtained by a processing of one or more plants of the following plant families: gourd (Cucurbitaceae), Papillonaceae, lindenlike plants (Tiliaceae), labiates (Labiatae), roots of white mallow (Althaeae radix), mallow leaves or blossoms (Marlae folium et flos), herbs of dyer's mallow (Marlae arboreae herba), Compositae or Asteraceae, Umbellifereae, Rutaceae, Chenopodiaceae, Linaceae, Rosaceae and Plantaginaceae, and which polysaccharide concentrates

g) in the carraghene oedema test on rat paws exhibit an anti-inflammatory activity which is comparable to that of 4-butyl-1,2-di-(phenyl)-pyrazolidine-3,5-dione (phenylbutazone) and of 1-[4'-(chloro)-benzoyl]-5-[methoxy]-2-[3''-

(methyl)-indolyl]-acetic acid (indometacine) and

h) their perorally determined $LD_{50}$ value with mice exceeds 3000 mg/kg body weight and with rats exceeds 2000 mg/kg body weight.

2. Polysaccharide concentrates according to claim 1, characterized in that they are obtained by a processing of one or more of the following plants or plant parts: chamomilla blossoms (Chamomillae flos), linden blossoms (Tiliae flos), plantain leaves (Plantaginia folium), fleabane seeds (Psyllii semen) (Pulicaria seeds), quince seeds or kernels (Cydoniae semen), linseed (Lini semen), Trigonella seeds (Trigonella foeni-graeci semen), gourd (Cucurbitae maximae fructus) and Kitaibella vitifoloa herba.

3. Polysaccharide concentrates according to claim 1 or 2, characterized in that they are obtained from gourd (Cucurbita maxima), their molecular weight exceeds 300,000, they contain nitrogen not in excess of 3% by weight, their residue on ignition is not in excess of 20% by weight, after an acid hydrolysis they contain mainly glucose, galactose and uronic acids, and in a dose of 10 mg/kg body weight they have in the carraghene oedema test on rat paws an anti-inflammatory activity of at least 50%.

4. Polysaccharide concentrates according to claim 1 or 2, characterized in that they are obtained from Trigonella seeds (Trigonella foeni-graeci semen), their molecular weight exceeds 100,000, they contain nitrogen not in excess of 3% by weight and phosphorus, expressed as phosphorus pentoxide, not in excess of 4% by weight, their residue on ignition is not in excess of 5% by weight, they contain at least 50% by weight reducing sugars and at least 70% by weight total sugars, after an acid hydrolysis they contain mainly galactose and mannose and in a dose of 10 mg/kg body weight they have in the carragheen oedema test on rat paws an anti-inflammatory activity of at least 40%.

5. Polysaccharide concentrates according to claims 1 to 4, characterized in that they are obtained in that the polysaccharide concentrate is precipitated by means of alcohol(s) used in 1 to 7 times the volume or acetone used in up to 10 times the volume of the aqueous extract or mono- to trivalent cations used in $1/20$ to $1/5$ of the weight of the drug plants.

6. Polysaccharide concentrates according to claims 1 to 5, characterized in that they are obtained from polysaccharide-containing drug plants which have been pretreated with one or more organic solvent(s).

7. Polysaccharide concentrates according to claims 1 to 6, characterized in that they are obtained from polysaccharide-containing drugs which have been treated with one or more aqueous organic solvent(s).

8. Polysaccharide concentrates according to claims 1 to 7, characterized in that they are obtained from polysaccharide-containing drugs which after a disintegration have been pretreated with the organic solvent(s).

9. Polysaccharide concentrates according to claims 1 to 8, characterized in that the treatment or extraction with the water is repeated.

10. Polysaccharide concentrates according to claims 1 to 9, characterized in that the treatment(s) and/or the extraction(s) with the water are performed after or during a disintegration.

11. Polysaccharide concentrates according to claims 1 to 10, characterized in that they are obtained in that water in a quantity corresponding to 1 to 20 times the weight of the drug plant is used for the treatment(s) with water.

12. Polysaccharide concentrates according to claims 1 to 11, characterized in that they are obtained in that the treatment or extraction with water is repeated.

13. Polysaccharide concentrates according to claims 1 to 12, characterized in that they are obtained in that the treatment(s) with the water and the separation of the solids are carried out after the disintegration of the fresh or dried drug plant(s) and the squeezing of the resulting pulp and comprise (another) treatment of the squeezed residue with water and another squeezing.

14. Polysaccharide concentrates according to claims 1 to 13, characterized in that they are obtained from one or more fresh or dried drug plant(s) which have been soaked in water.

15. Polysaccharide concentrates according to claims 1 to 14, characterized in that they are obtained in that the polysaccharide concentrate is precipitated by means of the alcohol(s), acetone and/or mono- to trivalent cations after the proteinlike substances have been separated and/or after an evaporation to a small volume.

16. Polysaccharide concentrates according to claims 1 to 15, characterized in that they are obtained in that the proteinlike substances contained in the aqueous extract are removed in that the aqueous extract is heated to 40 to 80 °C and the resulting precipitate is removed.

17. Polysaccharide concentrates according to claims 1 to 16, characterized in that they are recovered in that the protein precipitate is separated by centrifuging and/or filtering.

18. Polysaccharide concentrates according to claims 1 to 17, characterized in that they are obtained in that the volume of the alcohol(s) used to precipitate the polysaccharide concentrate is one to three times the volume of the aqueous extract.

19. Polysaccharide concentrates according to claims 1 to 18, characterized in that they are obtained in that the polysaccharide concentrate is separated from the aqueous phase by centrifuging and/or filtering after the mixture has been allowed to stand and the precipitate is subsequently washed with the precipitant.

20. Polysaccharides according to claims 1 to 19, characterized in that they are obtained in that the washing of the separated polysaccharide concentrate is repeated.

21. Polysaccharide concentrates according to claims 1 to 20, characterized in that they are obtained in that the resulting polysaccharide concentrate is purified.

22. Polysaccharide concentrates according to claims 1 to 21, characterized in that they are obtained in that the polysaccharide concentrates are purified by reprecipitation.

23. Polysaccharide concentrates according to claims 1 to 22, characterized in that they are obtained in that the reprecipitation is repeated.

24. Polysaccharide concentrates according to claims 1 to 23, characterized in that they are obtained in that the raw polysaccharide concentrate obtained by a single precipitation is purified by a reprecipitation in that it is dissolved in water and precipitated with a precipitant that can be used for the first precipitation, and the sequence of the additions of water and precipitant may be selected as desired.

25. Polysaccharide concentrates according to claims 1 to 24, characterized in that the raw polysaccharide obtained by a single precipitation is purified by a reprecipitation in that the concentrate is dissolved in water having 5 to 15 times, particularly ten times, the weight of the concentrate, and is heated to 65 to 80 °C, particularly 75 °C, the precipitates are removed by centrifugation, the polysaccharide concentrate is precipitated from the clear solution by an addition of a precipitant, particularly alcohol, which can be used for the first precipitation, the mixture is allowed to stand particularly for 1 to 24 hours, more particularly for 24 hours, and the precipitate is subsequently separated by centrifuging and/or filtering and is washed with the precipitant, particularly alcohol, and is dried under a vacuum at temperatures not in excess of 60 °C.

26. Polysaccharide concentrates according to claims 1 to 25, characterized in that they are obtained in that the reprecipitation is effected by means of an alcohol.

27. Polysaccharide concentrates according to claims 1 to 26, characterized in that they are obtained in that the reprecipitation is repeated once or several times.

28. Polysaccharide concentrates according to claims 1 to 27, characterized in that they are obtained in that the drying is effected at temperatures not in excess of 40 °C.

29. Polysaccharide concentrates according to claims 1 to 28, characterized in that they are obtained in that the purification or further purification of the raw or purified polysaccharide concentrate which has been obtained is carried out in such a manner that said concentrate is dissolved at 50 to 75 °C in water, which is used in a quantity that is 25 to 40 times, particularly 30 to 40 times, the weight of the concentrate, the solution is purified by centrifuging and is then forced under superatmospheric pressure through a membrane filter which is permeable to substances having a molecular weight under 75,000, and from the solution which has remained on the high-molecular side of the filter the polysaccharide is precipitated by means of a precipitant which can be used for the first precipitation.

30. Polysaccharide concentrates according to claims 1 to 29, characterized in that they are obtained in that the solution with has remained on the high-molecular side of the membrane filter is diluted with water before the polysaccharide is precipitated from said solution.

31. Polysaccharide concentrates according to claims 1 to 30, characterized in that it is obtained in that an alcohol is used to precipitate the polysaccharide from the solution that has remained on the high-molecular side of the membrane filter.

32. Polysaccharide concentrates according to claims 1 to 31, characterized in that they are obtained in that the dissolving in water and the purification by centrifuging and/or membrane filtration and/or precipitation are repeated once or several times.

33. Medicaments characterized by a content of one or more polysaccharide concentrate(s) according to claims 1 to 32 as active ingredients.

34. Medicaments according to claim 33, characterized in that they contain the polysaccharide concentrate(s) together with one or more other non-steroidal anti-inflammatory substances and/or one or more conventional pharmaceutical carrier(s) and/or adjuvant(s).

35. Cosmetic preparations characterized by a content of one or more polysaccharide concentrate(s) according to claims 1 to 32 as active ingredients.

36. Cosmetic preparations according to claim 35, characterized in that they contain the polysaccharide concentrate(s) together with one or more conventional, externally applicable carrier(s) and/or adjuvant(s).

**Claims for the Contracting State AT**

1. A process for preparing polysaccharide concentrates which are of vegetable origin and have
   a) a molecular weight from 75,000 to 2,000,000,
   b) a content of
   α) not in excess of 5% by weight nitrogen (microkjeldahl procedure in accordance with Wagner-Parnass; Hungarian standard 6830–60) and
   β) not in excess of 5% by weight phosphorus, expressed as phosphorus pentoxide (with molybdenate eiconogene reagent; Peac-Tracey: Moderne Methoden der Pflanzenanalyse, Volume IV, page 308, Springer-Verlag, 1955, Berlin),
   c) a residue on ignition not in excess of 25% by weight,
   d)
   α) at least 30% by weight reducing sugars (determined after an acid hydrolysis with o-toluidine; R. Richterich: Klinische Chemie, 1968, page 233, Carger Ed. Basel - New York) and
   β) a total sugar content of at least 60% by weight (determined by the phenol-sulphuric acid procedure; M. Dubois: Anal. Chem. 28 (1956), page 35),
   e) virtually no content of substances having molecular weights below 75,000 (examined with a molecular sieve consisting of a three-dimensionally cross-linked polysaccharide, which has been obtained by a cross-linking of the linear macromolecules of dextrane, and has an approximately medium degree of cross-linking) (Sephadex G 75*) and
   f) after acid hydrolysis contain at least two of the components glucose, galactose, xylose, rhamnose, arabinose and uronic acid(s), which polysaccharide concentrates have been obtained by a treatment of polysaccharide-containing drug plants with solvents for the polysaccharides in a process in which one or more fresh or dried drug plants, which contain polysaccharides, are treated or extracted with water at 0 to 40 °C for 1 to 24 hours and thereafter or during that time the respective solids are removed, followed by a precipitation of the polysaccharide concentrate from the liquid extract or extracts (liquid parts) by an addition of one or more alcohol(s) having one to three carbon atom(s), acetone and/or mono- to trivalent cations, whereafter the polysaccharide concentrate is separated from the aqueous phase, and which polysaccharide concentrates are characterized in that they are obtained by a processing of one or more plants of the following plant families: gourd (Cucurbitaceae), Papillonaceae, lindenlike plants (Tiliaceae), labiates (Labiatae), roots of white mallow (Althaeae radix), mallow leaves or blossoms (Marlae folium et flos), herbs of dyer's mallow (Marlae arboreae herba), Compositae or Asteraceae, Umbellifereae, Rutaceae, Chenopodiaceae, Linaceae, Rosaceae and Plantaginaceae, and which polysaccharide concentrates
   g) in the carraghene oedema test on rat paws exhibit an anti-inflammatory activity which is comparable to that of 4-butyl-1,2-di-(phenyl)-pyrazolidine-3,5-dione (phenylbutazone) and of 1-[4'-(chloro)-benzoyl]-5-[methoxy]-2-[3''-(methyl)-indolyl]-acetic acid (indometacine) and
   h) their perorally determined $LD_{50}$ value with mice exceeds 3000 mg/kg body weight and with rats exceeds 2000 mg/kg body weight, these polysaccharide concentrates being obtained by treatment of polysaccharide-containing, fresh or dried drug plant(s) is or are treated or extracted with water at 0 to 40 °C for 1 to 24 hours, whereafter the respective solids are removed, the polysaccharide concentrate is recovered from the aqueous extract or extracts (liquid part(s)) by an addition of one or more alcohol(s) which contain 1 to 3 carbon atom(s), acetone and/or mono- to trivalent cations, and the polysaccharide concentrate is separated from the aqueous phase, characterized in that they are prepared by processing of one or more plants of the following plant families: gourd (Cucurbitaceae), Papillonaceae, lindenlike plants (Tiliaceae), labiates (Labiatae), roots of white mallow (Althaeae radix), mallow leaves or blossoms (Marlae folium et flos), herbs of dyer's mallow (Marlae arboreae herba), Compositae or Asteraceae, Umbellifereae, Rutaceae, Chenopodiaceae, Linaceae, Rosaceae and Plantaginaceae.

2. A process according to claim 1, characterized in that the polysaccharide concentrates are obtained by processing of chamomilla blossoms (Chamomillae flos), linden blossoms (Tiliae flos), plantain leaves (Plantaginia folium), fleabane seeds (Psyllii semen) (Pulicaria seeds), quince seeds or kernels (Cydoniae semen), linseed (Lini semen), Trigonella seeds (Trigonella foeni-graeci semen), gourd (Cucurbitae maximae fructus) and Kitaibella vitifoloa herba.

3. A process according to claim 1 or 2, characterized in that the polysaccharide concentrates are obtained from gourd (Cucurbita maxima), their molecular weight exceeds 300,000, they contain nitrogen not in excess of 3% by weight, their residue on ignition is not in excess of 20% by weight, after an acid hydrolysis they contain mainly glucose, galactose and uronic acids, and in a dose of 10 mg/kg body weight they have in the carraghene oedema test on rat paws an anti-inflammatory activity of at least 50%.

4. A process according to claim 1 or 2, characterized in that the polysaccharide concentrates are obtained from Trigonella seeds (Trigonella foeni-graeci semen), their molecular weight exceeds 100,000, they contain nitrogen not in excess of 3% by weight and phosphorus, expressed as phosphorus pentoxide, not in excess of 4% by weight, their residue on ignition is not in excess of 5% by weight, they contain at least 50% by weight reducing sugars and at least 70% by weight total sugars, after an acid hydrolysis they contain mainly galactose and mannose and in a dose of 10 mg/kg body weight they have in the carragheen oedema test on rat paws an anti-inflammatory activity of at least 40%.

5. A process according to claims 1 to 4, characterized in that the polysaccharides are obtained in that the polysaccharide concentrate is precipitated by means of alcohol(s) used in 1 to 7 times the volume or acetone used in up to 10 times the volume of the aqueous extract or mono- to trivalent cations used in $1/20$ to $1/5$ of the weight of the drug plants.

6. A process according to the claims 1 to 5, characterized in that the polysaccharide concentrates are obtained from polysaccharide-containing drug plants which have been pretreated with one or more organic solvent(s).

7. A process according to the claims 1 to 6, characterized in that the polysaccharide concentrates are obtained from polysaccharide-containing drugs which have been treated with one or more aqueous organic solvent(s).

8. A process according to the claims 1 to 7, characterized in that the polysaccharide concentrates are obtained from polysaccharide-containing drugs which after a disintegration have been pretreated with the organic solvent(s).

9. A process according to the claims 1 to 8, characterized in that the polysaccharide concentrates are obtained in that the treatment or extraction with the water is repeated.

10. A process according to the claims 1 to 9, characterized in that the polysaccharide concentrates are obtained in that the treatment(s) and/or the extraction(s) with the water are performed after or during a disintegration.

11. A process according to the claims 1 to 10, characterized in that the polysaccharide concentrates are obtained in that water in a quantity corresponding to 1 to 20 times the weight of the drug plant is used for the treatment(s) with water.

12. A process according to the claims 1 to 11, characterized in that the polysaccharide concentrates are obtained in that the treatment or extraction with water is repeated.

13. A process according to the claims 1 to 12, characterized in that the polysaccharide concentrates are obtained in that the treatment(s) with the water and the separation of the solids are carried out after the disintegration of the fresh or dried drug plant(s) and the squeezing of the resulting pulp and comprise (another) treatment of the squeezed residue with water and another squeezing.

14. A process according to the claims 1 to 13, characterized in that the polysaccharide concentrates are obtained from one or more fresh or dried drug plant(s) which have been soaked in water.

15. A process according to the claims 1 to 14, characterized in that the polysaccharide concentrates are obtained in that the polysaccharide concentrate is precipitated by means of the alcohol(s), acetone and/or mono- to trivalent cations after the proteinlike substances have been separated and/or after an evaporation to a small volume.

16. A process according to the claims 1 to 15, characterized in that the polysaccharide concentrates are obtained in that the proteinlike substances contained in the aqueous extract are removed in that the aqueous extract is heated to 40 to 80 °C and the resulting precipitate is removed.

17. A process according to the claims 1 to 16, characterized in that the polysaccharide concentrates are obtained in that the protein precipitate is separated by centrifuging and/or filtering.

18. A process according to the claims 1 to 17, characterized in that the polysaccharide concentrates are obtained in that the volume of the alcohol(s) used to precipitate the polysaccharide concentrate is one to three times the volume of the aqueous extract.

19. A process according to the claims 1 to 18, characterized in that the polysaccharide concentrates are obtained in that the polysaccharide concentrate is separated from the aqueous phase by centrifuging and/or filtering after the mixture has been allowed to stand and the precipitate is subsequently washed with the precipitant.

20. A process according to the claims 1 to 19, characterized in that the polysaccharide concentrates are obtained in that the washing of the separated polysaccharide concentrate is repeated.

21. A process according to the claims 1 to 20, characterized in that the polysaccharide concentrates are obtained in that the resulting polysaccharide concentrate is purified.

22. A process according to the claims 1 to 21, characterized in that the polysaccharide concentrates are obtained in that the polysaccharide concentrates are purified by reprecipitation.

23. A process according to the claims 1 to 22, characterized in that the polysaccharide concentrates are obtained in that the reprecipitation is repeated.

24. A process according to the claims 1 to 23, characterized in that the polysaccharide concentrates are obtained in that the raw polysaccharide concentrate obtained by a single precipitation is purified by a reprecipitation in that it is dissolved in water and precipitated with a precipitant that can be used for the first precipitation, and the sequence of the additions of water and precipitant may be selected as desired.

25. A process according to the claims 1 to 24, characterized in that the polysaccharide concentrates are obtained in that the raw polysaccharide obtained by a single precipitation is purified by a reprecipitation in that the concentrate is dissolved in water having 5 to 15 times, particularly ten times, the weight of the concentrate, and is heated to 65 to 80 °C, particularly 75 °C, the precipitates are removed by centrifugation, the polysaccharide concentrate is precipitated from the clear solution by an addition of a precipitant, particularly alcohol, which can be used for the first precipitation, the mixture is allowed to stand particularly for 1 to 24 hours, more particularly for 24 hours, and the precipitate is subsequently separated by centrifuging and/or filtering and is washed with the precipitant, particularly alcohol, and is dried under a vacuum at temperatures not in excess of 60 °C.

26. A process according to the claims 1 to 25, characterized in that the polysaccharide concentrates are obtained in that the reprecipitation is effected by means of an alcohol.

27. A process according to the claims 1 to 26, characterized in that the polysaccharide concentrates are obtained in that the reprecipitation is repeated once or several times.

28. A process according to the claims 1 to 27, characterized in that the polysaccharide concentrates are obtained in that the drying is effected at temperatures not in excess of 40 °C.

29. A process according to the claims 1 to 28, characterized in that the polysaccharide concentrates are obtained in that the purification or further purification of the raw or purified polysaccharide concentrate which has been obtained is carried out in such a manner that said concentrate is dissolved at 50 to 75 °C in water, which is used in a quantity that is 25 to 40 times, particularly 30 to 40 times, the weight of the concentrate, the solution is purified by centrifuging and is then forced under superatmospheric pressure through a membrane filter which is permeable to substances having a molecular weight under 75,000, and from the solution which has remained on the high-molecular side of the filter the polysaccharide is precipitated by means of a precipitant which can be used for the first precipitation.

30. A process according to the claims 1 to 29, characterized in that the polysaccharide concentrates are obtained in that the solution which has remained on the high-molecular side of the membrane filter is diluted with water before the polysaccharide is precipitated from said solution.

31. A process according to the claims 1 to 30, characterized in that the polysaccharide concentrates are obtained in that an alcohol is used to precipitate the polysaccharide from the solution that has remained on the high-molecular side of the membrane filter.

32. A process according to the claims 1 to 31, characterized in that the polysaccharide concentrates are obtained in that the dissolving in water and the purification by centrifuging and/or membrane filtration and/or precipitation are repeated once or several times.

33. A process for preparing a medicament, characterized in that one or more polysaccharide concentrate(s) according to claims 1 to 32 as active ingredients are mixed with one or more conventional pharmaceutical carrier(s) and/or adjuvant(s).

34. A process according to claim 33, characterized in that the polysaccharide concentrate(s) are mixed with one or more other non-steroidal anti-inflammatory substances and/or one or more conventional pharmaceutical carrier(s) and/or adjuvant(s).

35. Cosmetic preparations characterized by a content of one or more polysaccharide concentrate(s) according to claims 1 to 32 as active ingredients.

36. Cosmetic preparations according to claim 35, characterized in that they contain the polysaccharide concentrate(s) together with one or more conventional, externally applicable carrier(s) and/or adjuvant(s).

**Revendications pour les Etats contractants BE, CH, DE, FR, GB, IT, LI, LU, NL, SE**

1. Concentrés de polysaccharides d'origine végétale ayant
   a) un poids moléculaire de 75 000 à 2 000 000,
   b) une teneur en
   α) azote de 5% en poids au maximum (mode opératoire en microkjeldahl selon Wagner-Parnass; norme hongroise 6830–66) et
   β) phosphore de 5% au maximum, exprimé en pentoxyde de phosphore, (avec le réactif au molybdénate-eiconogène; Peac-Tracey: Moderne Methoden der Pflanzenanalyse, tome IV, page 308, Springer-Verlag, 1955, Berlin), qui
   c) abandonnent 25% en poids au maximum de résidus de calcination,
   d)
   α) contiennent au moins 30% en poids de sucres réducteurs (déterminés à l'o-toluidine après hydrolyse acide; R. Richterich: Klinische Chemie 1968, page 233, Carger Ed. Bâle-New-York) et
   β) dont la teneur en sucres totale s'élève au moins à 60% en poids (déterminée selon le mode opératoire au phénol-acide sulfurique; M. Dubois: Anal. Chem. 28 [1956], page 35), étant entendu
   e) qu'ils ne contiennent pratiquement aucune substance de poids moléculaires au-dessous de 75 000 (examinés au moyen d'un tamis moléculaire, constitué par un polysaccharide tridimensionnel réticulé qui a été obtenu par réticulation transversale des macromolécules linéaires de dextrane et qui a un degré de réticulation sensiblement moyen), et
   f) qu'ils contiennent, après hydrolyse acide, au moins 2 des constituants, glucose, galactose, xylose, rhamnose, arabinose et acides(s) uronique(s), obtenus par traitement de plantes médicinales contenant des polysaccharides avec des agents dissolvant les polysaccharides, en traitant ou respectivement en extrayant avec de l'eau pendant 1 à 24 heures à 0–40 °C, une ou plusieurs plantes médicinales contenant des polysaccharides, fraîches ou séchées, et en éliminant ensuite ou pendant cette opération [suivant le cas] les substances solides, puis en précipitant le concentré de polysaccharides à partir du ou des macérat(s) ou extrait(s) aqueux {fraction(s) liquide(s)} par addition de 1 ou plusieurs alcool(s) à 1 à 3 atome(s) de carbone, d'acétone et/ou de cations 1- à 3-valents et en séparant ledit concentré de polysaccharides de la phase aqueuse, caractérisés en ce qu'ils sont préparés par traitement de 1 ou plusieurs plantes des familles de plantes suivantes: courge (Cucurbitaceae), papilionacées (Papilionaceae), végétaux de l'espèce tilleul (Tiliaceae), labiées (Labiatae), racines de guimauve (Althaea radix), feuille ou fleur de mauve (Malvae folium et flos), herbe de mauve colorante (Malvae arboreae herba), composées (Compositae ou Asteraceae), ombellifères (Umbellifereae), végétaux de la famille des rutacées (Rutaceae), végétaux de la famille de l'ansérine (Chenopodiaceae), végétaux de l'espèce lin (Linaceae), végétaux de la famille des rosiers (Rosaceae) et végétaux de l'espèce plantain (Plantaginaceae), ces concentrés de polysaccharides

g) présentant, dans l'essai de l'oedème à la carraghénine sur la patte du rat, une activité anti-inflammatoire comparable à l'activité de la 4-butyl-1,2-di-(phényl)-pyrazolidine-3,5-dione {phénylbutazone}
et de l'acide
1-[4'-(chloro)-benzoyl]-[méthoxy]-2[3''-(méthyl)-indolyl]-acétique {indométacine} et

h) leur valeur $DL_{50}$ déterminée par voie orale chez la souris étant supérieure à 3 000 mg/kg de poids du corps et chez le rat étant supérieure à 2 000 mg/kg de poids du corps.

2. Concentrés de polysaccharides selon la revendication 1, caractérisés en ce qu'ils sont préparés par traitement de 1 ou plusieurs des plantes ou parties de plantes suivantes:
fleur de camomille (Chamomillae flos), fleur de tilleul (Tiliae flos), feuille de plantain (Plantaginis folium), graine d'herbe à puce (Psyllii semen), graine ou pépin de coing (Cydoniae semen), graine de lin (Lini semen), graine de fenugrec (Trigonella foeni-graeci semen), courge (Cucurbitae maximae fructus) et Kitaibela vitifolia herba.

3. Concentrés de polysaccharides selon la revendication 1 ou 2, caractérisés en ce qu'ils sont préparés à partir de courge (Cucurbita maxima), que leur poids moléculaire est supérieur à 300 000, qu'ils contiennent au maximum 3% en poids d'azote, abandonnent au maximum 20% en poids de résidus de calcination et contiennent d'après l'hydrolyse acide surtout du glucose, du galactose et des acides uroniques, et qu'à une dose de 10 mg/kg de poids du corps leur activité anti-inflammatoire dans l'essai de l'oedème à la carraghénine sur la patte de rat, s'élève au minimum à 50%.

4. Concentrés de polysaccharides selon la revendication 1 ou 2, caractérisés en ce qu'ils sont préparés à partir de graines de fenugrec (Trigonella foeni-graeci semen), que leur poids moléculaire est supérieur à 100 000, qu'ils contiennent au maximum 3% en poids d'azote et au maximum 4% en poids de phosphore, exprimés en pentoxyde de phosphore, abandonnent au maximum 5% en poids de résidus de calcination, contiennent au moins 50% en poids de sucres réducteurs et au moins 70% en poids de sucres totaux et contiennent d'après l'hydrolyse acide surtout du galactose et du mannose, et qu'à une dose de 10 mg/kg de poids du corps leur activité anti-inflammatoire dans l'essai de l'oedème à la carraghénine sur la patte de rat s'élève au minimum à 40%.

5. Concentrés de polysaccharides selon les revendications 1 à 4, caractérisés en ce qu'ils sont préparés de telle façon que l'on utilise pour la précipitation du concentré de polysaccharides, le ou les alcool(s) à raison de 1 à 7 fois en volume et l'acétone jusqu'à 10 fois en volume, dans chaque ces par rapport au macérat ou extrait aqueux, et les cations 1- à 3-valents en quantité du $1/20$ème au $1/5$ème, par rapport au poids des plantes médicinales.

6. Concentrés de polysaccharides selon les revendications 1 à 5, caractérisés en ce qu'ils sont préparés en utilisant des plantes médicinales, contenant des polysaccharides, qui ont été préalablement traitées par 1 ou plusieurs solvant(s) organique(s).

7. Concentrés de polysaccharides selon les revendications 1 à 6, caractérisés en ce qu'ils sont préparés en utilisant des plantes médicinales, contenant des polysaccharides, pour le prétraitement desquelles est ou sont utilisé(s) comme solvant(s) organique(s) un ou des solvants organiques aqueux.

8. Concentrés de polysaccharides selon les revendications 1 à 7, caractérisés en ce qu'ils sont préparés en utilisant des plantes médicinales, contenant des polysaccharides, dont le prétraitement avec le ou les solvant(s) organique(s) est effectué après une désintégration.

9. Concentrés de polysaccharides selon les revendications 1 à 8, caractérisés en ce qu'ils sont préparés de telle façon que l'on effectue le traitement ou l'extraction plusieurs fois avec de l'eau.

10. Concentrés de polysaccharides selon les revendications 1 à 9, caractérisés en ce qu'ils sont préparés de telle façon que l'on effectue le(s) traitement(s) ou la ou les extraction(s) avec de l'eau après une ou pendant une désintégration.

11. Concentrés de polysaccharides selon les revendications 1 à 10, caractérisés en ce qu'ils sont préparés de telle façon que l'on utilise pour le ou les traitement(s) avec de l'eau, la quantité d'eau de 1 à 20 fois, par rapport au poids de la plante médicinale.

12. Concentrés de polysaccharides selon les revendications 1 à 11, caractérisés en ce qu'ils sont préparés de telle façon que l'on effectue le traitement ou l'extraction avec de l'eau, plusieurs fois.

13. Concentrés de polysaccharides selon les revendications 1 à 12, caractérisés en ce qu'ils sont préparés de façon que l'on effectue le ou les traitement(s) avec de l'eau et la séparation des substances solides d'une manière telle qu'après la désintégration de la ou des plante(s) médicinale(s) fraîche(s) ou de la ou des plante(s) médicinale(s) séchée(s) et l'expression de la suspension obtenue, on traite [de nouveau] avec de l'eau le résidu de pressage et on l'exprime de nouveau.

14. Concentrés de polysaccharides selon les revendications 1 à 13, caractérisés en ce qu'ils sont préparés de façon que l'on utilise comme plante(s) médicinale(s) fraîche(s) ou séchée(s), une ou de telle(s) plante(s) arrosées d'eau.

15. Concentrés de polysaccharides selon les revendications 1 à 14, caractérisés en ce qu'ils sont préparés de façon que l'on effectue la précipitation du concentré de polysaccharides avec le ou les alcool(s), l'acétone et/ou les cations 1- à 3-valents après séparation des substances de type protéinique et/ou concentration à un faible volume.

16. Concentrés de polysaccharides selon les revendications 1 à 15, caractérisés en ce qu'ils sont préparés de façon que l'on effectue l'élimination des substances de type protéinique se trouvant dans le macérat ou l'extrait aqueux, de telle manière que l'on chauffe le macérat ou extrait aqueux à 40–80 °C et on sépare le dépôt ayant précipité.

17. Concentrés de polysaccharides selon les revendications 1 à 16, caractérisés en ce qu'ils sont préparés de façon que l'on effectue la séparation du dépôt protéinique par centrifugation et/ou filtration.

18. Concentrés de polysaccharides selon les revendications 1 à 17, caractérisés en ce qu'ils sont obtenus de façon que pour la précipitation du concentré de polysaccharides, on utilise le ou les alcool(s) à raison de 1 à 3 fois en volume, par rapport au macérat ou extrait aqueux.

19. Concentrés de polysaccharides selon les revendications 1 à 18, caractérisés en ce qu'ils sont préparés de façon qu'on effectue la séparation du concentré de polysaccharides à partir de la phase aqueuse après abandon au repos, par centrifugation et/ou filtration, après quoi on le lave avec l'agent de précipitation.

20. Concentrés de polysaccharides selon les revendications 1 à 19, caractérisés en ce qu'ils sont préparés de façon que l'on effectue plusieurs fois le lavage du concentré de polysaccharides séparé.

21. Concentrés de polysaccharides selon les revendications 1 à 20, caractérisés en ce qu'ils sont obtenus de façon que l'on purifie le concentré de polysaccharides obtenu.

22. Concentrés de polysaccharides selon les revendications 1 à 21, caractérisés en ce qu'ils sont préparés de façon que l'on effectue la purification du concentré de polysaccharides par reprécipitation.

23. Concentrés de polysaccharides selon les revendications 1 à 22, caractérisés en ce qu'ils sont préparés de façon que l'on effectue la reprécipitation plusieurs fois.

24. Concentrés de polysaccharides selon les revendications 1 à 23, caractérisés en ce qu'ils sont préparés de façon que l'on effectue la purification par reprécipitation du concentré de polysaccharides brut précipité une première fois, de telle manière qu'on le dissout dans l'eau et on le précipite avec un agent de précipitation utilisable pour la première précipitation, la succession d'addition de l'eau et de l'agent de précipitation pouvant être choisie dans un ordre quelconque.

25. Concentrés de polysaccharides selon les revendications 1 à 24, caractérisés en ce qu'ils sont préparés de façon que l'on effectue la purification par reprécipitation du concentré de polysaccharides brut précipité une première fois, de telle manière qu'on le dissout dans la quantité de 5 à 15 fois d'eau, par rapport à son poids, et on le chauffe à 65–80 °C, on élimine par centrifugation les substances ayant précipité, on précipite à partir de la solution limpide le concentré de polysaccharides par addition d'un agent précipitant utilisable pour la première précipitation, on abandonne le mélange au repos, puis on sépare par centrifugation et/ou filtration le dépôt, on le lave avec l'agent précipitant et on le sèche sous vide à 60 °C au maximum.

26. Concentrés de polysaccharides selon les revendications 1 à 25, caractérisés en ce qu'ils sont préparés de façon que l'on effectue la reprécipitation avec un alcool.

27. Concentrés de polysaccharides selon les revendications 1 à 26, caractérisés en ce qu'ils sont préparés de façon que l'on répète une ou plusieurs fois la reprécipitation.

28. Concentrés de polysaccharides selon les revendications 1 à 27, caractérisés en ce qu'ils sont préparés de façon qu'on effectue le séchage à 40 °C au maximum.

29. Concentrés de polysaccharides selon les revendications 1 à 28, caractérisés en ce qu'ils sont préparés de façon que l'on effectue la purification ou la purification ultérieure du concentré de polysaccharides brut ou purifié obtenu, de telle manière qu'on le dissout dans la quantité de 25 à 40 fois d'eau, par rapport à son poids, à 50–75 °C, on purifie la solution par centrifugation, puis on la presse sous un excès de pression à travers un filtre à membrane laissant passer les substances ayant un poids moléculaire au-dessous de 75 000 et on précipite le polysaccharide à partir de la solution subsistant du côté des poids moléculaires élevés du filtre à membrane, avec un agent précipitant utilisable pour la première précipitation.

30. Concentrés de polysaccharides selon les revendications 1 à 29, caractérisés en ce qu'ils sont préparés de façon que l'on dilue avec de l'eau la solution subsistant du côté des poids moléculaires élevés du filtre à membrane avant la précipitation du polysaccharide.

31. Concentrés de polysaccharides selon les revendications 1 à 30, caractérisés en ce qu'ils sont préparés de façon que l'on effectue la précipitation du polysaccharide à partir de la solution subsistant du côté des poids moléculaires élevés du filtre à membrane, avec un alcool.

32. Concentrés de polysaccharides selon les revendications 1 à 31, caractérisés en ce qu'ils sont préparés de façon que l'on répète une fois ou plusieurs fois la dissolution dans l'eau et la purification par centrifugation et/ou filtration sur membrane et/ou précipitation.

33. Médicaments, caractérisés par une teneur en un ou plusieurs concentré(s) de polysaccharides selon les revendications 1 à 32, en tant que substances actives.

34. Médicaments selon la revendication 33, caractérisés en ce qu'ils contiennent le ou les concentré(s) de polysaccharides conjointement avec une ou plusieurs autre(s) substance(s) anti-inflammatoire(s) non-stéroïde(s) et/ou un ou plusieurs véhicule(s) et/ou adjuvant(s) pharmaceutiquement courants.

35. Compositions cosmétiques, caractérisées par une teneur en un ou plusieurs concentré(s) de polysaccharides selon les revendications 1 à 32, en tant que substances actives.

36. Compositions cosmétiques selon la revendication 35, caractérisées en ce qu'elles contiennent le ou les concentré(s) de polysaccharides conjointement avec un ou plusieurs véhicule(s) et/ou adjuvant(s) utilisables par voie externe courants.


**Revendications pour l'Etat contractant AT**

1. Procédé pour préparation des concentrés de polysaccharides d'origine végétale ayant
   a) un poids moléculaire de 75 000 à 2 000 000,
   b) une teneur en
   α) azote de 5% en poids au maximum (mode opératoire en microkjeldahl selon Wagner-Parnass, norme hongroise 6830–66) et
   β) phosphore de 5% au maximum, exprimé en pentoxyde de phosphore, (avec le réactif au molybdénate-eiconogène; Peac-Tracey: Moderne Methoden der Pflanzenanalyse, tome IV, page 308, Springer-Verlag, 1955, Berlin), qui
   c) abandonnent 25% en poids au maximum de résidus de calcination,
   d)
   α) contiennent au moins 30% en poids de sucres réducteurs (déterminés à l'o-toluidine après hydrolyse acide; R. Richterich: Klinische Chemie 1968, page 233, Carger Ed. Bâle-New-York) et
   β) dont la teneur en sucres totale s'élève au moins à 60% en poids (déterminée selon le mode opératoire au phénol-acide sulfurique; M. Dubois: Anal. Chem. 28 [1956], page 35), étant entendu
   e) qu'ils ne contiennent pratiquement aucune substance de poids moléculaires au-dessous de 75 000 (examinés au moyen d'un tamis moléculaire, constitué par un polysaccharide tridimensionnel réticulé qui a été obtenu par réticulation transversale des macromolécules linéaires de dextrane et qui a un degré de réticulation sensiblement moyen), et
   f) qu'ils contiennent, après hydrolyse acide, au moins 2 des constituants, glucose, galactose, xylose, rhamnose, arabinose et acide(s) uronique(s), obtenus par traitement de plantes médicinales contenant des polysaccharides avec des agents dissolvant les polysaccharides, en traitant ou respectivement en extrayant avec de l'eau pendant 1 à 24 heures à 0–40°C, une ou plusieurs plantes médicinales contenant des polysaccharides, fraîches ou séchées, et en éliminant ensuite ou pendant cette opération [suivant le cas] les substances solides, puis en précipitant le concentré de polysaccharides à partir du ou des macérat(s) ou extrait(s) aqueux {fraction(s) liquide(s)} par additon de 1 ou plusieurs alcool(s) à 1 à 3 atome(s) de carbone, d'acétone et/ou de cations 1- à 3-valents et en séparant ledit concentré de polysaccharides de la phase aqueuse, caractérisés en ce qu'ils sont préprés par traitement de 1 ou plusieurs plantes des familles de plantes suivantes: courge (Cucurbitaceae), papilionacées (Papilionaceae), végétaux de l'espèce tilleul (Tiliaceae), labiées (Labiatae) racines de guimauve (Althaearadix), feuille ou fleur de mauve (Malvae folium et flos), herbe de mauve colorante (Malvae arboreae herba), composées (Compositae ou Asteraceae), ombellifères (Umbellifereae), végétaux de la famille des rutacées (Rutaceae), végétaux de la famille de l'ansérine (Chenopodiaceae), végétaux de l'espèce lin (Linaceae), végétaux de la famille des rosiers (Rosaceae) et végétaux de l'espèce plantain (Plantaginaceae), ces concentrés de polysaccharides
   g) présentant, dans l'essai de l'oedème à la carraghénine sur la patte du rat, une activité anti-inflammatoire comparable à l'activité de la 4-butyl-1,2-di-(phényl)-pyrazolidine-3,5-dione {phénylbutazone}
et de l'acide 1-[4'-(chloro)-benzoyl]-[méthoxy]-2[3''-(méthyl)-indolyl]-acétique {indométacine} et
   h) leur valeur $DL_{50}$ déterminée par voie orale chez la souris étant supérieure à 3 000 mg/kg de poids du corps et chez le rat étant supérieure à 2 000 mg/kg de poids du corps, ces concentrés de polysaccharides sont préparés par traitement de plantes médicinales contenant des polysaccharides avec des agents dissolvant les polysaccharides, en traitant ou respectivement en extrayant avec de l'eau pendant 1 à 24 heures à 0–40°C, 1 ou plusieurs plantes médicinales contenant des polysaccharides, fraîches ou séchées, et on élimine ensuite ou pendant cette opération [suivant le cas] les substances solides, puis on précipite le concentré de polysaccharides à partir du ou des macérat(s) ou extrait(s) aqueux {fraction(s) liquide(s)} par addition de 1 ou plusieurs alcool(s) à 1 à 3 atome(s) de carbone, d'acétone et/ou de cations 1- à 3-valents et on sépare ledit concentré de polysaccharides de la phase aqueuse, caractérisé en ce qu'ils sont préparés par traitement de 1 ou plusieurs plantes des familles de plantes suivantes: courge (Cucurbitaceae), papilionacées (Papilionaceae), végétaux de l'espèce tilleul (Tiliaceae), labiées (Labiatae), racines de guimauve (Althaearadix), feuille ou fleur de mauve (Malvae folium et flos), herbe de mauve colorante (Malvae arboreae herba), composées (Compositae ou Asteraceae), ombellifères (Umbellifereae), végétaux de la famille des rutacées (Rutaceae), végétaux de la famille de l'ansérine (Chenopodiaceae), végétaux de l'espèce lin (Linaceae), végétaux de la famille des rosiers (Rosaceae) et végétaux de l'espèce plantain (Plantaginaceae).

2. Procédé selon la revendication 1, caractérisé en ce que les concentrés de polysaccharides sont préparés par traitement de 1 ou plusieurs des plantes ou parties de plantes suivantes: fleur de camomille (Chamomillae flos), fleur de tilleul (Tiliae flos), feuille de plantain (Plantaginis folium), graine d'herbe à puce (Psyllii semen), graine ou pépin de coing (Cydoniae semen), graine de lin (Lini semen), graine de fenugrec (Trigonella foeni-graeci semen), courge (Cucurbitae maximae fructus) et Kitaibela vitifolia herba.

3. Procédé selon la revendication 1 ou 2, caractérisé en ce que les concentrés de polysaccharides sont préparés à partir de courge (Cucurbita maxima), que leur poids moléculaire est supérieur à 300 000, qu'ils contiennent au maximum 3% en poids d'azote, abandonnent au maximum 20% en poids de résidus de calcination et contiennent d'après l'hydrolyse acide surtout du glucose, du galactose et des acides uroniques, et qu'à une dose de 10 mg/kg de poids du corps leur activité anti-inflammatoire dans l'essai de l'oedème à la carraghénine sur la patte de rat, s'élève au minimum à 50%.

4. Procédé selon la revendication 1 ou 2, caractérisé en ce que les concentrés de polysaccharides sont préparés à partir de graines de fenugrec (Trigonella foeni-graeci semen), que leur poids moléculaire est supérieur à 100 000, qu'ils contiennent au maximum 3% en poids d'azote et au maximum 4% en poids de phosphore, exprimés en pentoxyde de phosphore, abandonnent au maximum 5% en poids de résidus de calcination, contiennent au moins 50% en poids de sucres réducteurs et au moins 70% en poids de sucres totaux et contiennent d'après l'hydrolyse acide surtout du galactose et du mannose, et qu'à une dose de 10 mg/kg de poids du corps leur activité anti-inflammatoire dans l'essai de l'oedème à la carraghénine sur la patte de rat s'élève au minimum à 40%.

5. Procédé selon les revendications 1 à 4, caractérisé en ce que les concentrés de polysaccharides sont préparés de telle façon que l'on utilise pour la précipitation du concentré de polysaccharides, le ou les alcool(s) à raison de 1 à 7 fois en volume et l'acétone jusqu'à 10 fois en volume, dans chaque ces par rapport au macérat ou extrait aqueux, et les cations 1- à 3-valents en quantité du $1/20$ème au $1/5$ème, par rapport au poids des plantes médicinales.

6. Procédé selon les revendications 1 à 5, caractérisé en ce que les concentrés de polysaccharides sont préparés en utilisant des plantes médicinales, contenant des polysaccharides, qui ont été préalablement traitées par 1 ou plusieurs solvant(s) organique(s).

7. Procédé selon les revendications 1 à 6, caractérisé en ce que les concentrés de polysaccharides sont préparés en utilisant des plantes médicinales, contenant des polysaccharides, pour le prétraitement desquelles est ou sont utilisé(s) comme solvant(s) organique(s) un ou des solvants organiques aqueux.

8. Procédé selon les revendications 1 à 7, caractérisé en ce que les concentrés de polysaccharides sont préparés en utilisant des plantes médicinales, contenant des polysaccharides, dont le prétraitement avec le ou les solvant(s) organique(s) est effectué après une désintégration.

9. Procédé selon les revendications 1 à 8, caractérisé en ce que les concentrés de polysaccharides sont préparés de telle façon que l'on effectue le traitement ou l'extraction plusieurs fois avec de l'eau.

10. Procédé selon les revendications 1 à 9, caractérisé en ce que les concentrés de polysaccharides sont préparés de telle façon que l'on effectue le(s) traitement(s) ou la ou les extraction(s) avec de l'eau après une ou pendant une désintégration.

11. Procédé selon les revendications 1 à 10, caractérisé en ce que les concentrés de polysaccharides sont préparés de telle façon que l'on utilise pour le ou les traitement(s) avec de l'eau, la quantité d'eau de 1 à 20 fois, par rapport au poids de la plante médicinale.

12. Procédé selon les revendications 1 à 11, caractérisé en ce que les concentrés de polysaccharides sont préparés de telle façon que l'on effectue le traitement ou l'extraction avec de l'eau, plusieurs fois.

13. Procédé selon les revendications 1 à 12, caractérisé en ce que les concentrés de polysaccharides sont préparés de façon que l'on effectue le ou les traitement(s) avec de l'eau et la séparation des substances solides d'une manière telle qu'après la désintégration de la ou des plante(s) médicinale(s) fraîche(s) ou de la ou des plante(s) médicinale(s) séchée(s) et l'expression de la suspension obtenue, on traite [de nouveau] avec de l'eau le résidu de pressage et on l'exprime de nouveau.

14. Procédé selon les revendications 1 à 13, caractérisé en ce que les concentrés de polysaccharides sont préparés de façon que l'on utilise comme plante(s) médicinale(s) fraîche(s) ou séchée(s), une ou de telle(s) plante(s) arrosées d'eau.

15. Procédé selon les revendications 1 à 14, caractérisé en ce que les concentrés de polysaccharides sont préparés de façon que l'on effectue la précipitation du concentré de polysaccharides avec le ou les alcool(s), l'acétone et/ou les cations 1- à 3-valents après séparation des substances de type protéinique et/ou concentration à un faible volume.

16. Procédé selon les revendications 1 à 15, caractérisé en ce que les concentrés de polysaccharides sont préparés de façon que l'on effectue l'élimination des substances de type protéinique se trouvant dans le macérat ou extrait aqueux, de telle manière que l'on chauffe le macérat ou extrait aqueux à 40–80 °C et on sépare le dépôt ayant précipité.

17. Procédé selon les revendications 1 à 16, caractérisé en ce que les concentrés de polysaccharides sont préparés de façon que l'on effectue la séparation du dépôt protéinique par centrifugation et/ou filtration.

18. Procédé selon les revendications 1 à 17, caractérisé en ce que les concentrés de polysaccharides sont obtenus de façon que pour la précipitation du concentré de polysaccharides, on utilise le ou les alcool(s) à raison de 1 à 3 fois en volume, par rapport au macérat ou extrait aqueux.

19. Procédé selon les revendications 1 à 18, caractérisé en ce que les concentrés de polysaccharides sont préparés de façon qu'on effectue la séparation du concentré de polysaccharides à partir de la phase aqueuse après abandon au repos, par centrifugation et/ou filtration, après quoi on le lave avec l'agent de précipitation.

20. Procédé selon les revendications 1 à 19, caractérisé en ce que les concentrés de polysaccharides sont préparés de façon que l'on effectue plusieurs fois le lavage du concentré de polysaccharides séparé.

21. Procédé selon les revendications 1 à 20, caractérisé en ce que les concentrés de polysaccharides sont obtenus de façon que l'on purifie le concentré de polysaccharides obtenu.

22. Procédé selon les revendications 1 à 21, caractérisé en ce que les concentrés de polysaccharides sont préparés de façon que l'on effectue la purification du concentré de polysaccharides par reprécipitation.

23. Procédé selon les revendications 1 à 22, caractérisé en ce que les concentrés de polysaccharides sont préparés de façon que l'on effectue la reprécipitation plusieurs fois.

24. Procédé selon les revendications 1 à 23, caractérisé en ce que les concentrés de polysaccharides sont préparés de façon que l'on effectue la purification par reprécipitation du concentré de polysaccharides brut précipité une première fois, de telle manière qu'on le dissout dans l'eau et on le précipite avec un agent de précipitation utilisable pour la première précipitation, la succession d'addition de l'eau et de l'agent de précipitation pouvant être choisie dans un ordre quelconque.

25. Procédé selon les revendications 1 à 24, caractérisé en ce que les concentrés de polysaccharides sont préparés de façon que l'on effectue la purification par reprécipitation du concentré de polysaccharides brut précipité une première fois, de telle manière qu'on le dissout dans la quantité de 5 à 15 fois d'eau, par rapport à son poids, et on le chauffe à 65–80°C, on élimine par centrifugation les substances ayant précipité, on précipite à partir de la solution limpide le concentré de polysaccharides par addition d'un agent précipitant utilisable pour la première précipitation, on abandonne le mélange au repos, puis on sépare par centrifugation et/ou filtration le dépôt, on le lave avec l'agent précipitant et on le sèche sous vide à 60°C au maximum.

26. Procédé selon les revendications 1 à 25, caractérisé en ce que les concentrés de polysaccharides sont préparés de façon que l'on effectue la reprécipitation avec un alcool.

27. Procédé selon les revendications 1 à 26, caractérisé en ce que les concentrés de polysaccharides sont préparés de façon que l'on répète une ou plusieurs fois la reprécipitation.

28. Procédé selon les revendications 1 à 27, caractérisé en ce que les concentrés de polysaccharides sont préparés de façon qu'on effectue le séchage à 40°C au maximum.

29. Procédé selon les revendications 1 à 28, caractérisé en ce que les concentrés de polysaccharides sont préparés de façon que l'on effectue la purification ou la purification ultérieure du concentré de polysaccharides brut ou purifié obtenu, de telle manière qu'on le dissout dans la quantité de 25 à 40 fois d'eau, par rapport à son poids, à 50–75°C, on purifie la solution par centrifugation, puis on la presse sous un excès de pression à travers un filtre à membrane laissant passer les substances ayant un poids moléculaire au-dessous de 75 000 et on précipite le polysaccharide à partir de la solution subsistant du côté des poids moléculaires élevés du filtre à membrane, avec un agent précipitant utilisable pour la première précipitation.

30. Procédé selon les revendications 1 à 29, caractérisé en ce que les concentrés de polysaccharides sont préparés de façon que l'on dilue avec de l'eau la solution subsistant du côté des poids moléculaires élevés du filtre à membrane avant la précipitation du polysaccharide.

31. Procédé selon les revendications 1 à 30, caractérisé en ce que les concentrés de polysaccharides sont préparés de façon que l'on effectue la précipitation du polysaccharide à partir de la solution subsistant du côté des poids moléculaires élevés du filtre à membrane, avec un alcool.

32. Procédé selon les revendications 1 à 31, caractérisé en ce que les concentrés de polysaccharides sont préparés de façon que l'on répète une fois ou plusieurs fois la dissolution dans l'eau et la purification par centrifugation et/ou filtration sur membrane et/ou précipitation.

33. Procédé pour la préparation d'un médicament, caractérisé en ce qu'un ou plusieurs concentré(s) de polysaccharides selon les revendications 1 à 32, en tant que substances actives sont mélangées avec un ou plusieurs véhicule(s).

34. Procédé selon la revendication 33, caractérisé en ce que le ou les concentré(s) de polysaccharides sont mélangés avec un ou plusieurs véhicule(s) et/ou adjuvant(s) pharmaceutiquement courants.

35. Compositions cosmétiques, caractérisées par une teneur en un ou plusieurs concentré(s) de polysaccharides selon les revendications 1 à 32, en tant que substances actives.

36. Compositions cosmétiques selon la revendication 35, caractérisées en ce qu'elles contiennent le ou les concentré(s) de polysaccharides conjointement avec un ou plusieurs véhicule(s) et/ou adjuvant(s) utilisables par voie externe courants.